# EUROPEAN PATENT APPLICATION

(11) **EP 1 081 225 A1**
(43) Date of publication of application: **07.03.2001**
(21) Application number: 99116766.9
(22) Date of filing: 30.08.1999
(51) Int. Cl.: C12N 15/12, C12N 5/10, C12N 1/21, C12N 1/19, C07K 14/47, A01K 67/027, A61K 49/00, C12Q 1/68

(54) **Transgenic animal model for neurodegenerative diseases**

(71) Applicant: Biofrontera Pharmaceuticals GmbH, 51377 Leverkusen (DE)
(72) Inventor: Lübbert, Hermann, Prof. Dr., 51381 Leverkusen (DE)
(74) Representative: Sternagel, Fleischer, Godemeyer & Partner Patentanwälte

(57) **Abstract**

The present application refers to a mouse parkin2 DNA- and protein sequence, containing mutations or deletions causing Parkinson's disease in a human if occurring in the according human sequence, the construction of a transgenic non-human animal containing such a mutated DNA sequence and therefore expressing no or a less active or non-active parkin protein as well as the use of this transgenic animal as a model for neurodegenerative diseases.

## Description

The present invention relates to a mouse parkin2 DNA- and protein sequence containing naturally occurring or artificially introduced mutations or deletions, which cause Parkinson's disease in a human if they occur in the according human sequence, the construction of a truncated parkin gene, which expresses no, a non-active or a truncated parkin protein and a model of a transgenic animal, expressing such a less or non-active parkin protein instead of the native parkin protein or no parkin protein, as well as to the use of such a transgenic animal as a model for neurodegenerative diseases, preferred Parkinson's disease.

Neurodegenerative disorders are some of the most feared illnesses in society. During the last 10 years some of the genetic causes of many of the primary neurodegenerative disorders like Alzheimer's disease, Parkinson's disease, Huntington's disease, amyotrophic lateral sclerosis, prion disease and several ataxic syndromes, have been identified. These findings gave new insults in the knowledge about the initiating trigger as well as the resulting consequences of those diseases. Due to the fact that these diseases have many pathological mechanisms in common it seems possible that only relatively few pathways to neuronal death are involved in these disorders. Thus, treatment strategies for a particular neurodegenerative disease may be found to have value in other related disorders.

Parkinson's disease is a progressive neurodegenerative movement disorder with severe symptoms like rigidity, bradykinesia or tremor. The disease symptoms appear after degeneration of more than 70-80% of dopaminergic neurons. Broadly speaking the disease falls into two categories, namely late onset and early onset. Late onset, which occurs in older age (55+ years), mainly as consequence of environmental influences, leads to enhanced dopaminergic neuron death at a faster rate and to a more severe degree than normal. Early onset Parkinson's disease is much more infrequent but starts between the ages of 35 and 60 years. There is evidence that three forms of this early type of Parkinson's disease show a tendency to run in families and is therefore known as familial Parkinson's disease.

In both the early and late onset types of Parkinson's disease, the pathology is the same but the abnormalities tend to be more severe and more widespread in cases beginning at an earlier age. The disease is characterised by lesions in brain areas where the cell bodies of the dopaminergic neurons are located mainly in the substantia nigra compacta. In addition intracytoplasmic inclusions known as Lewy bodies can be observed in different brain regions, in particular in substantia nigra and the locus ceruleus.

Recently two loci could be identified associated with early onset PD, one on human chromosome 4q21-23 ("PARK 1" gene locus) with a gene defect to be due to a missense mutation in the α-synuclein protein (or *parkin1),* a small abundant brain molecule (Polymeropoulos, M. *et al.,* Science 1997; 276:2045-2047), and one on chromosome 2p13 ("PARK 3" gene locus)(Gasser, *T. et al.,* Nat. Genet. 1998; 18: 262-265). Both forms are inherited in an autosomal dominant manner.

Lately an autosomal recessive form of familial Parkinson's disease could be observed, linked to human chromosome 6q25.2-27 ("PARK 2" gene locus) (Matsumine, H. *et al.,* Am J Hum Genet (1997); 60: 588-596). This gene, designated *parkin* (or later *parkin2)* contains 12 exons spanning more than 500 kb and encodes a protein of 465 amino acids (molecular weight 51,652 Dalton) with homology to ubiquitin at the N-terminal portion and a RING-finger like motif at the C-terminal portion.

It has been shown, that mutations in the α-synuclein gene lead to autosomal dominant Parkinson's disease (Polymeropoulos, M.. *et al.,* Science 1997; 276: 2045-2047), as well as mutations in the parkin gene cause autosomal recessive juvenile parkinsonism (Kitada, *T. et al.,* Nature 1998; 392: 605.608; Hattori, N. *et al.,* Biochem Biophys Res Comm 1998; 249: 754-758)).

Further Hattori, N. *et al.,* have been shown in Ann Neurol 1998; 44: 935-941, that different deletions in the parkin gene are the reason for truncated parkin proteins, causing autosomal recessive juvenile parkinsonism. Especially intragenic deletional mutations, involving exons 3 to 4, exon 3, exon 4 and exon 5, as well as exon 3 through exon 7 are described as effecting the disease. Deletion of exon 3 of the parkin gene is furthermore described by Lücking, C. *et al.* in the Lancet 1998; 352: 1355-1356 to cause autosomal recessive juvenile parkinsonism. Investigations of Abbas, N. *et al.* Human Molecular Genetics 1999; 8: 567-574 and Kitada, T. *et al.,* Nature 1998; 392: 805-808 show that mutations in the ubiquitin-like N-terminal part (exon 2) of the parkin gene can also cause autosomal recessive juvenile parkinsonism, as well as different frameshift- or missense mutations.

Leroy, E. *et al.,* demonstrated in Hum Genet 1998; 103: 424-427 that deletions of exons 5, 6 and 7 of the human parkin gene leads to early onset Parkinson's disease.

At present most common therapies are dealing with the increase of dopamine content in PD patients via application of L-dopa as precursor of dopamine, dopamine agonists or MAO-B (Monoamino Oxidase B) inhibitors, e.g. Deprenyl, by blocking the degradation of dopamine. There are no prophylactic therapies available to stop the progression of the degenerative disease before onset of symptoms in late onset PD. This is due to the fact that at present diagnosis is only possible when first symptoms occur. So far it is not clear to which extent genetic components enhance the environmental components responsible for the increased cell death of dopaminergic neurons.

Although different transgenic animal models for neurodegenerative diseases like Alzheimer's disease have been created, a transgenic animal model for Parkinson's disease has not yet been described.

Homologous recombination may be employed for inactivation or alteration of genes in a site-directed manner. A number of papers describe the use of homologous recombination in mammalian cells, including human cells. Illustrative of these papers are Kucherlpati *et al.* (1984) Proc. Natl. Acad. Sci. USA 81:3153-3157; Kucherlapati *et al.* (1985) Mol. Cell. Bio. 5:714-720; Smithies *et al.* (1985) Nature 317:230-234; Wake *et al.* (1985) Mol. Cell. Bio. 8:2080-2089; Ayares *et al.* (1985) Genetics 111:375-388; Ayares *et al.* (1986) Mol. Cell. Bio. 7:1656-1662; Song *et al.* (1987) Proc. Natl. Acad. Sci. USA 84:6820-6824; Thomas *et al.* (1986) Cell 44:419-428; Thomas and Capecchi (1987) Cell 51:503-512; Nandi *et al.* (1988) Proc. Natl. Acad. Sci. USA 85:3845-3849; and Mansour *et al.* (1988) Nature 336:348-352. Various aspects of using homologous recombination to create specific genetic mutations in embryonic stem cells and to transfer these mutations to the germline have been described (Evans and Kaufman (1981) Nature 294:154-146; Dotschman *et al.,* (1987) Nature 330:576-578; Thomas and Capecchi (1987) Cell 51:503-512; Thompson *et al.* (1989) Cell 56:316-321. The combination of a mutant polyoma enhancer and a thymidine kinase promoter to drive the neomycin gene has been shown to be active in both embryonic stem cells and EC cells by Thomas and Capecchi, supra, 1987; Nicholas and Berg (1983) in Teratocarcinoma Stem Cell, eds. Siver, martin and Strikland (Cold Spring Harbor Lab., Cold Spring Harbor, N.Y. (pp. 469-497); and Linney and Donerly, Cell 35:693-699, 1983.

The object of the present application is to provide the suppositions for a test model for neurodegenerative diseases, preferably Parkinson's disease and a valuable tool in the diagnosis and treatment of these conditions, as well as the development of experimental models of Parkinson's disease that can be used to define further the underlying biochemical events involved in the pathogenesis of this disease.

This object is met by a polynucleotide sequence encoding a mouse parkin2 protein, containing naturally occurring or artificially introduced mutations or deletions, which cause Parkinson's disease in a human if they occur in the according human sequence, a vector, containing such a sequence, a prokaryotic or eukaryotic cell, containing such a vector and a transgenic non-human animal, whose one or both alleles of a gene encoding a parkin gene are mutated in a way, that a protein with modified, preferred less activity or no active protein is expressed.

The transgenic non-human animals according to the present invention can be used as models for analysing the symptoms of neurodegenerative diseases or as a model system for testing the efficacy of a treatment for a neurodegenerative disease, whereby it is not an object of the present application to provide any method for treating one of the described diseases in a human or animal.

Such models could presumably be employed, in one application, to screen for agents that alter the degenerative course of Parkinson's disease. For example, a model system of Parkinson's disease could be used to screen for environmental factors that induce or accelerate the pathogenesis. Further an experimental model could be used to screen for agents that inhibit, prevent, or reverse the progression of Parkinson's disease. Presumably, such models could be employed to develop pharmaceuticals that are effective in preventing, arresting, or reversing Parkinson's disease. Further such models can be used for examination of behaviour during the development of a neurodegenerative disease, for examination of physiological and molecular biological correlation of the disease, for studies of drug effects and for determination of effective drug doses and toxicity. These applications should be considered as examples and should not limit the application of the models in any way.

The present invention provides model systems of neurodegenerative diseases, preferred Parkinson's disease, wherein the model system comprises a mutated isoform or a fragment of the mouse parkin2 gene (further designated as *mPark2),* a DNA sequence derived from SEQ ID NO: 1 encoding a mouse parkin2 protein corresponding to the human parkin protein encoded by human chromosome gene region 6q25.2-27 ("PARK 2" gene locus). Preferred the model system contains a mutated mPark2 sequence or a mPark2 sequence containing any deletion, coding for a mutated or truncated, less active or non-active parkin protein.

The sequence of human α-synuclein *(parkin1)* gene, as well as human parkin *(parkin2)* gene is known. Human parkin2 gene (further designated as *hPark2)* contains 12 exons, coding for a protein which has in full length 465 amino acids and a molecular weight of 51,652 Daltons.

The present application shows the full length cDNA of mPark2 in SEQ ID NO:1, consisting of 12 exons, containing the full length open reading frame for the mouse parkin2 protein (SEQ ID NO:4) which coding region consists of 1395 bp, coding for a protein of 464 amino acids with a calculated molecular weight of 51615 Dalton. Further two shorter cDNAs spanning a coding region of 789 bp (SEQ ID NO: 2 (isolated from mouse brain cDNA library by specific PCR)) and 753 bp (SEQ ID NO: 3 (isolated from mouse kidney cDNA library by specific PCR)) corresponding to amino acid sequences of 262 amino acids (SEQ ID NO:5) and 250 amino acids (SEQ ID NO:6) respectively are provided.

During the work of isolation and sequencing of the sequences SEQ ID NO: 1 to 3 shown in this application Shimizu, N. *et al.* submitted a mouse parkin DNA sequence to the EMBL GenBank database, published in July 1999 with the accession number AB019558. The protein sequence of the mouse parkin protein encoded by the published sequence is identical to SEQ ID No: 4.

The present invention refers to polynucleic acid sequences derived from SEQ ID NO: 1, containing naturally occurring or artificially introduced mutations or deletions, which are known to cause Parkinson's disease in a human if they occur in the according human sequence.

The present invention encompasses further polynucleotide sequences containing naturally occurring mutations according to the wobble principle, which represents the degeneration of the genetical code, as well as according to the polymorphism of the genetical code, encoding any protein which has the same or a homologous amino acid sequence as any of the mutated or truncated mouse parkin2 proteins of the present invention.

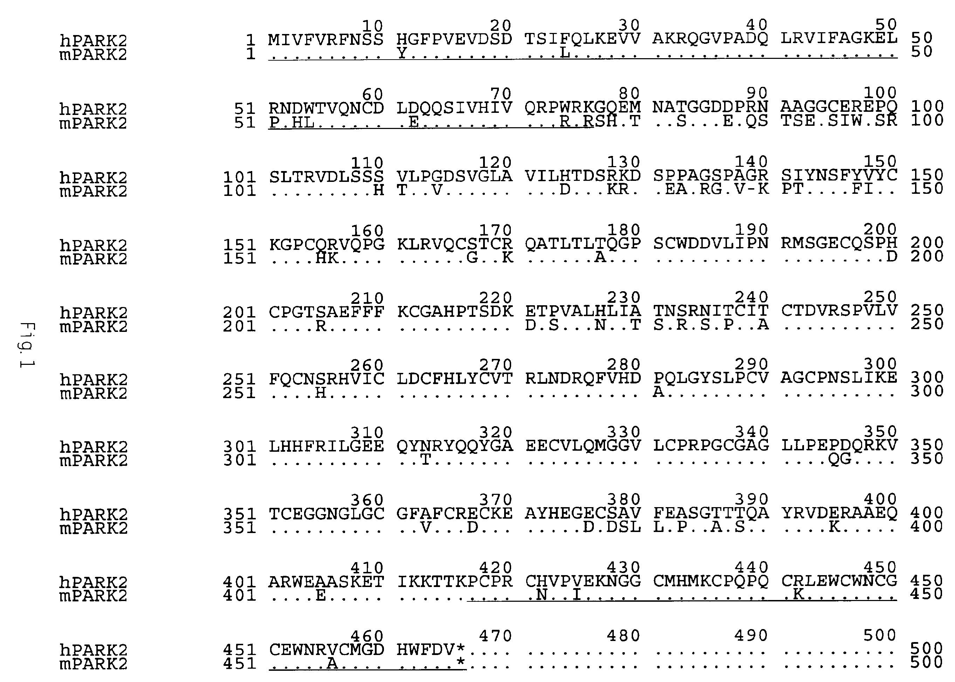

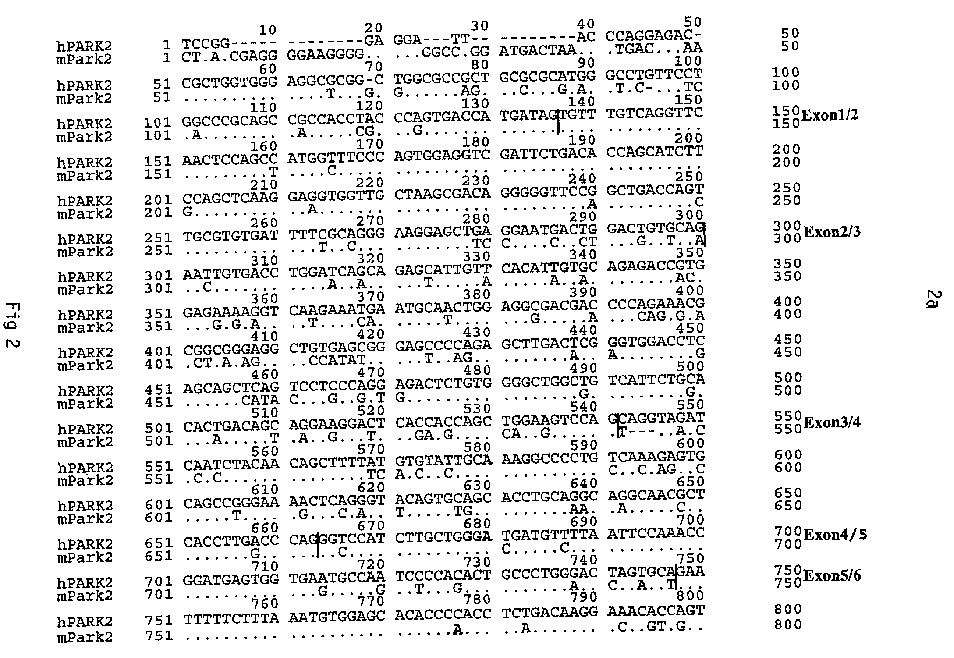

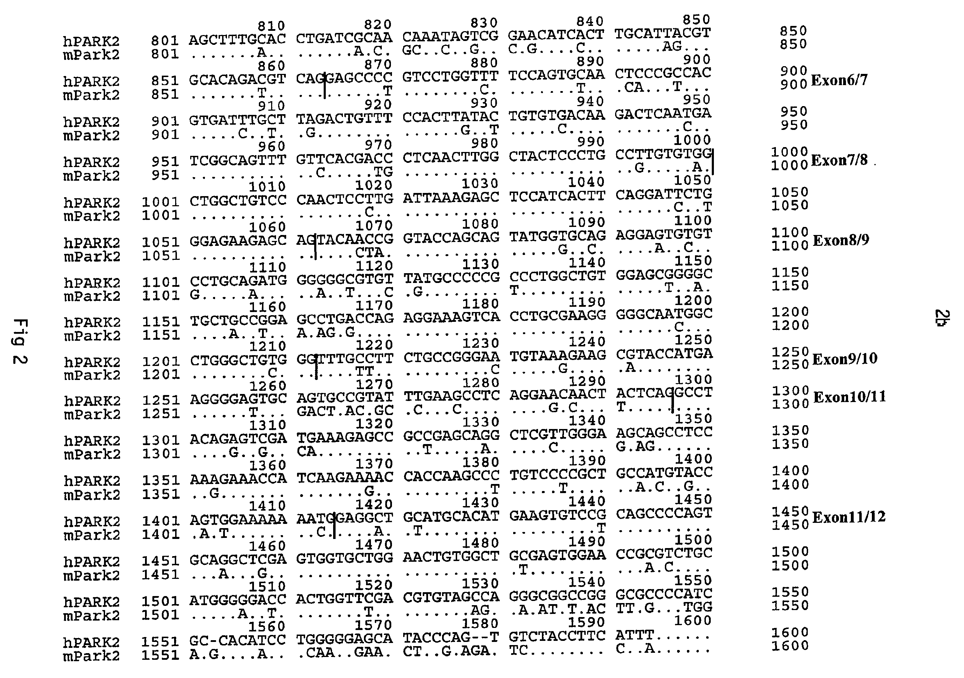

"Homologous amino acid sequence" in content with the mouse parkin2 protein means in the present application an amino acid sequence, wherein at least 70 %, preferably 80 %, more preferably 90 % of the amino acids are identical to one of the proteins of the present invention and wherein the replaced amino acids preferably are replaced by homologous amino acids. As "homologous" amino acids are designated which have similar features concerning hydrophobicity, charge, steric features etc. Most preferred are amino acid sequences, containing the species-dependent differences of the mouse amino acid sequence compared to human parkin protein shown in the alignment Figure No. 1. The alignment of the corresponding polynucleotide sequences with the exon boundaries is shown in Figure No. 2.

In the whole application for nucleotides and amino acids the usual designations (one-letter ore three-letter code) are used, known by any person skilled in the art.

The full length polynucleotide sequence of SEQ ID NO:1 or fragments thereof can be obtained by isolation of genomic DNA, containing exons and introns of the mPark2 gene, by RNA transcripts of the DNA or by the preparation of cDNA, containing only the exons of the mPark2 gene. Further the full length sequence as well as fragments thereof may be obtained by synthetical polymerisation of nucleotides.

A preferred polynucleotide sequence of the present application is a polynucleotide sequence derived from SEQ ID NO: 1, which is either mutated or in which parts of the sequence are deleted. Mutations, insertions or deletions may be located 5'upstream of the open reading frame (i.e. in the promotor-region), or they can concern one or more exons of the open reading frame. More preferred is a sequence, containing either a mutated full length sequence or fragments of SEQ ID NO:1, encoding a truncated parkin2 protein (i.e. by mutations leading to a STOP codon or by deletions) or no protein (i.e. if the mutation or deletion is located in the promoter-region in exon 1).

More preferred mutations or deletions concern either exon 1, wherein the promotor region is contained, or exon 3 and/or one or more of the other exons.

Most preferred the polynucleotide sequence of the present application is selected from SEQ ID NO: 2, SEQ ID NO: 3, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17; SEQ ID NO: 18, SEQ ID NO:19 or SEQ ID NO:20 (see also Table 1 and 2).

One of the polynucleotide sequences SEQ ID NO:1 to 3 may be treated *in vitro* or *in vivo* by random or site-directed mutagenesis, by random or site-directed digestion, by recombination or fusion or any other method known of persons skilled in the art to obtain sequences derived from SEQ ID NO:1 containing mutations or deletions leading to a less active or to no parkin protein. Of course a person skilled in the art will understand that the present invention encompasses as well any construction in which parts of or the whole polynucleotide sequence encoding the parkin gene is deleted or replaced by another sequence (i.e. by a sequence encoding an antibioticum-resistance).

To obtain at least a transgenic non-human animal as a model for neurodegenerative diseases, the natural occurring sequence of the parkin gene in this animal may be replaced on one or both alleles of the chromosomes by a sequence of mPark2, containing mutations or deletions according to the present invention. These animals produce either less or less active or no parkin protein.

The transgenic animals of the present invention are created using targeted gene replacement, a sequence by which a specific DNA sequence of interest (target DNA) is replaced by an altered DNA (replacement DNA). The genome of embryonic stem (ES) cells is modified using homologous recombination (Capecchi, Science 1989: 244:1288 and U.S. Pat. No. 5,487,992). The embryonic stem cells are injected in blastocysts as an early state of the developing embryo. The blastocysts are then placed in a pseudopregnant female animal.

Briefly, a vector is constructed that carries the replacement DNA. Both ends of the replacement DNA are flanked by long DNA sequences homologous to the sequences flanking the target DNA. When the vector is introduced into ES cells, the homologous sequences align and recombination may take place. This results in the target DNA being exchanged for the replacement DNA. The vector is not replicated in the cells and will be lost. The frequency of homologous recombination is low; thus, a screening system is used. The replacement DNA will contain a positive marker sequence, usually a neomycin resistance gene. Thus, any cells that incorporate the replacement DNA by homologous recombination will resist neomycin. By growing cells in medium containing the drug neomycin one can select only those cells containing the replacement DNA. The ES cells containing the replacement DNA are then inserted into recipient mouse blastocysts to create chimeric mice. Chimeras with germ cells derived for the altered ES cells transmit the modified genome to their offspring, yielding mice heterozygous for the target DNA (contain one target DNA and one replacement DNA). The heterozygotes are then bred with each other either to create mice homozygous for the replacement DNA and deficient in the target DNA or to maintain transgenic heterozygotes if the homozygotic mice are not viable.

The DNA will comprise at least a portion of the gene(s) at the particular locus with introduction of a lesion into at least one, usually both copies, of the native gene(s), so as to prevent expression of a functional parkin protein. The lesion may be an insertion, deletion, replacement or combination thereof. When the lesion is introduced into only one copy of the gene being inactivated, the (heterozygote) cells having a single unmutated copy of the target gene are amplified and may be subjected to a second transformation, where the lesion may be the same or different from the first lesion, usually different, and where a deletion, or replacement is involved, may be overlapping at least a portion of the lesion originally introduced. The resulting transformants are screened for the absence of the functional protein of interest and the DNA of the cell may be further screened to ensure the absence of a wild-type target gene. Alternatively, homozygosity as to a phenotype may be achieved by breeding hosts heterozygous for the mutation.

For the construction of a transgenic animal model according to the present application any suitable animal may be employed, however mammals are preferred. More preferred are rodents and most preferred are rats and mice.

In the following the single steps of creating the animal models will be described in detail.

Starting from a polynucleotide sequence encoding a parkin gene, preferably from a sequence encoding a mPark2 gene, more preferably from a sequence according to any of SEQ ID NO:1 to 3, most preferred from SEQ ID NO: 1 a desired mutation, insertion or deletion is introduced to the sequence. Methods to create mutations by random or site-directed mutagenesis or desired insertions or deletions by random or site-directed digestion and/or replacement are commonly known to persons skilled in the art and broadly described in the literature. The method how a mutation, insertion or deletion is introduced in the sequence is not relevant, however falls under the scope of the present invention, as long as any of the later described nucleotides, amino acids or sequences are involved.

The constructs may be modified to include functional entities other than the mutated sequence which may find use in the preparation of the construct, amplification, transformation of the host cell, and integration of the construct into the host cell.

The homologous sequence for targeting the construct may have one or more deletions, insertions, substitutions or combinations thereof. For example, the mPark2 gene may include a deletion at one site and an insertion at another site which includes a gene which may be used for selection, where the presence of the inserted gene will result in a defective inactive protein product. Preferably, substitutions are employed. For an inserted gene, of particular interest is a gene which provides a marker, e.g., antibiotic resistance such as neomycin resistance, including G418 resistance.

The deletion will be at least about 50 bp, or more usually at least about 100 bp, and generally not more than about 20 kbp, where the deletion will normally include at least a portion of the coding region including a portion of or one or more exons, a portion of one or more introns, and may or may not include a portion of the flanking non-coding regions, particularly the 5'-non-coding region (transcriptional regulatory region). Thus, the homologous region may extend beyond the coding region into the 5'-non-coding region or alternatively into the 3'-non-coding region. Insertions will generally not exceed 10 kbp, usually not exceed 5 kbp, generally being at least 50 bp, more usually at least 200 bp.

The homologous sequence should include at least about 100 bp, preferably at least about 150 bp, more preferably at least about 300 bp of the target sequence and generally not exceeding 20 kbp, usually not exceeding 10 kbp, preferably less than about a total of 5 kbp, usually having at least about 50 bp on opposite sides of the insertion and/or the deletion in order to provide for double crossover recombination.

Upstream and/or downstream from the target gene construct may be a gene which provides a tool to select out primary random integration of the construct in the genome. For this purpose, the herpes simplex virus thymidine kinase gene may be employed, since the presence of the thymidine kinase gene may be detected by the use of nucleoside analogs, such as Gancyclovir or Acyclovir, for their cytotoxic effects on cells that contain a functional HSV-tk gene. The absence of sensitivity to these nucleoside analogs indicates that homologous recombination has occurred.

The presence of the marker gene inserted into the gene of interest establishes the integration of the target construct into the host genome.

However, DNA analysis might be required in order to establish whether homologous or non-homologous recombination occurred. This can be determined by employing probes for the insert and then sequencing the 5' and 3' regions flanking the insert for the presence of the gene of interest extending beyond the flanking regions of the construct or identifying the presence of a deletion, when such deletion is introduced.

The polymerase chain reaction (PCR) may be used, with advantage in detecting the presence of homologous recombination (Kim and Smithies, (1988) Nucleic Acid Res. 16:8887-8903; and Joyner et al (1989) Nature 338:153-156). Primers may be used which are complementary to a sequence within the construct, usually complementary to the selection marker gene, and complementary to a sequence outside the construct and at the target locus. In this way, one can only obtain DNA duplexes having both of the primers present in the complementary chains in homologous recombination has occurred. By demonstrating the presence of the primer sequences or the expected size sequence, the occurrence of homologous recombination is supported. Any person skilled in the art knows how to determine the suitable PCR primers and conditions.

The construct may further include a replication system which is functional in the mammalian host cell. For the most part, these replication systems will involve viral replication systems, such as Simian Virus 40, Epstein-Barr virus, papilloma virus, adenovirus and the like.

Where a marker gene is involved, as an insert, and/or flanking gene, depending upon the nature of the gene, it may have the wild-type transcriptional regulatory regions, particularly the transcriptional initiation regulatory region or a different transcriptional initiation region. Whenever a gene is from a host where the transcriptional initiation region is not recognized by the transcriptional machinery of the mammalian host cell, a different transcriptional initiation region will be required. This region may be constitutive or inducible, preferably inducible. A wide variety of transcriptional initiation regions have been isolated and used with different genes. Of particular interest as promoters are the promoters of metallothionein-I and II from a mammalian host, thymidine kinase, beta-actin, immunoglobulin promoter, human cytomegalovirus promoters, and SV40 promoters. In addition to the promoter, the wild-type enhancer may be present or an enhancer from a different gene may be joined to the promoter region.

The construct may further include a replication system for prokaryotes, particularly E. coli, for use in preparing the construct, cloning after each manipulation, allowing for analysis, such as restriction mapping or sequencing, followed by expansion of a clone and isolation of the plasmid for further manipulation. When necessary, a different marker may be employed for detecting bacterial transformants.

Once the vector has been prepared, it may be further manipulated by deletion of the bacterial sequences as well as linearisation, where a short deletion may be provided in the homologous sequence, generally not exceeding about 500 bp, generally being from about 50 to 300 bp. The small deletion will generally be near one or other end of the targeted structural gene.

The construction of the desired polynucleotide sequence may be carried out in a cloning vector and linearised prior to the transfection of ES cells. A broad range of cloning vectors as well as vectors for the homologous recombination are commercially available and may be selected according to the desired construction.

Cloning vectors are usually replicated in prokaryotic cells, which renders the selection and multiplication of the desired construct. It is not critical which prokaryotic organism is used, but usually E.coli or a yeast strain is preferred.

E. coli is one prokaryotic host useful particularly for cloning the DNA sequences of the present invention. Other microbial hosts suitable for use include bacilli, such as Bacillus subtilus, and other enterobacteriaceae, such as Salmonella, Serratia, and various Pseudomonas species. In these prokaryotic hosts, one can also make expression vectors, which will typically contain expression control sequences compatible with the host cell (e.g., an origin of replication). In addition, any number of a variety of well-known promoters will be present, such as the lactose promoter system, a tryptophan (trp) promoter system, a beta-lactamase promoter system, or a promoter system from phage lambda. The promoters will typically control expression, optionally with an operator sequence, and have ribosome binding site sequences and the like, for initiating and completing transcription and translation.

Other microbes, such as yeast, may also be used for expression. Saccharomyces is a preferred host, with suitable vectors having expression control sequences, such as promoters, including 3-phosphoglycerate kinase or other glycolytic enzymes, and an origin of replication, termination sequences and the like as desired.

Homologous recombination may be used to insert a mutant sequence into a host genome at a specific site, for example, at a host parkin locus. In one type of homologous recombination, one or more host sequence(s) are replaced; for example, a host parkin allele (or portion thereof) is replaced with a mutant parkin allele (or portion thereof). In addition to such gene replacement methods, homologous recombination may be used to target a mutant parkin allele to a specific site other than a host parkin locus. Homologous recombination may be used to produce transgenic non-human animals and/or cells that incorporate mutant parkin alleles.

Further to the above described techniques a step of expressing the treated sequence may be inserted in the expiration. Therefore the construct is (sub)cloned into any expression vector, which may be brought into a suitable eukaryotic cell. These expression vectors are typically replicable in the host organisms either as episomes or as an integral part of the host chromosomal DNA. Commonly, expression vectors will contain selection markers, e.g., tetracycline resistance or hygromycin resistance, to permit detection and/or selection of those cells transformed with the desired DNA sequences. Polynucleotides encoding a variant parkin2 polypeptide may include sequences that facilitate transcription (expression sequences) and translation of the coding sequences, such that the encoded polypeptide product is produced. Construction of such polynucleotides is well known in the art and is described further in Maniatis et al. Molecular Cloning: A Laboratory Manual, 2nd Ed. (1989), Cold Spring Harbor, N.Y. For example, but not for limitation, such polynucleotides can include a promoter, a transcription termination site (polyadenylation site in eukaryotic expression hosts), a ribosome binding site, and, optionally, an enhancer for use in eukaryotic expression hosts, and, optionally, sequences necessary for replication of a vector.

Any suitable eukaryotic cell may be used, but insect cells or mammalian cells as primary cells or immortalized cell lines are preferred.

A number of suitable host cell lines capable of secreting intact human proteins have been developed in the art, and include the CHO cell lines, various COS cell lines, HeLa cells, myeloma cell lines, Jurkat cells, etc. Baculovirus expression systems are useful for high level expression of heterologous genes in eukaryotic cells. Knops *et al.* (1991) J. Biol. Chem. 266(11):7285. Expression vectors for these cells can include expression control sequences, such as an origin of replication, a promoter, an enhancer (Queen et al. (1986) Immunol. Rev. 89:49, and necessary processing information sites, such as ribosome binding sites, RNA splice sites, polyadenylation sites, and transcriptional terminator sequences. Preferred expression control sequences are promoters derived from immunoglobulin genes, SV40, Adenovirus, Bovine Papilloma Virus, and the like. The vectors containing the DNA segments of interest can be transferred into the host cell by well-known methods, which vary depending on the type of cellular host. For example, calcium chloride transfection is commonly utilized for prokaryotic cells, whereas calcium phosphate treatment, microinjection of DNA into the nucleus or electroporation may be used for other cellular hosts. (See, generally, Maniatis, et al. Molecular Cloning: A Laboratory Manual, 2^{nd}. Ed. Cold Spring Harbor Press, (1989). The DNA may be single or double stranded, linear or circular, relaxed or supercoiled DNA. For various techniques for transforming mammalian cells, see Keown et al., Methods in Enzymology (1990) 185:527-537.

For the creation of an animal model according to the present invention each polynucleotide sequence can be used, containing mutations, insertions or deletions which are known to cause Parkinson's disease in a human, when they occur in the corresponding human sequence. Preferred polynucleotide sequences for the creation of an animal model according to the present invention are those which mutations are shown in table 2. More preferred are polynucleotide sequences containing mutations or deletions shown in table 1. The most preferred polynucleotide sequence for the construction of a transgenic animal of the present invention is SEQ ID NO: 7 Further enclosed to the present invention is an animal model wherein the parkin sequence is replaced by an according sequence of another mammal (i.e. by the human sequence, containing one of the mutations, insertions or deletions described in the present application) or by a sequence encoding a marker, i.e. an antibioticum.

**Table 1:**

| Mutations or deletions in mPark2 cDNA (SEQ ID NO:1) | | | | |
|---|---|---|---|---|
| Position in SEQ ID NO:1 | Replacement (DNA) | Replacement (protein) | SEQ ID NO (DNA seq) | SEQ ID NO (prot seq) |
| NT 300-540 | Exon3 | Frameshift, Truncation | 7 | 21 |
| NT 300-659 | Exon3-4 | ORF, deletion of 121 aa | 8 | 22 |
| NT 300-996 | Exon3-7 | Frameshift, Truncation | 9 | 23 |
| NT 541-659 | Exon 4 | Frameshift, Truncation | 10 | 24 |
| NT 659-744 | Exon 5 | Frameshift, Truncation | 11 | 25 |
| NT 660-996 | Exon 5-7 | Frameshift, Truncation | 12 | 26 |
| NT 996-1208 | Exon 8-9 | Frameshift, Truncation | 13 | 27 |
| NT: 229-230 (aa 34) | deletion AG | Gln→Stop at aa 38, nonsense | 14 | 28 |
| NT: 282 (aa 52) | deletion A | Asn→Stop at aa 54, nonsense | 15 | 29 |
| NT: 350-351 (aa 74) | deletion AG | Arg→Stop at aa 78, nonsense | 16 | 30 |
| NT: 136-299 | Exon 2 | Frameshift, Truncation | 17 | 31 |

| | | | | |
|---|---|---|---|---|
| aa = amino acid | | | | |
| NT = nucleotide | | | | |

**Table 2:**

| Replaced amino acids in mPark2 cDNA (SEQ ID NO:1) | | | | |
|---|---|---|---|---|
| Position in SEQ ID NO:1 | Replacement (DNA) | Replacement (protein) | SEQ ID NO (DNA seq) | SEQ ID NO (prot seq) |
| NT: 608 | G→T, | Lys→Asn (aa 161) | 18 | 32 |
| NT: 1369 | C→A, | Thr→Asn (aa 415) | 19 | 33 |
| NT: 1483 | G→A, | Trp→Stop (aa 453) | 20 | 34 |

| | | | | |
|---|---|---|---|---|
| aa = amino acid | | | | |
| NT = nucleotide | | | | |

Once the construct has been prepared and manipulated, the DNA is isolated from the procaryotic host according to any method known in the art. Before the DNA construct is introduced into the target cells for homologous recombination undesired sequences may be removed from the vector, e.g. the undesired bacterial sequences. As target cells an embryonic stem (ES) cell line may be used. As already indicated above for the expression system, any convenient technique for introducing the DNA into the target cells may be employed. After transformation of the target cells, many target cells are selected by means of positive and/or negative markers, as previously indicated, neomycin resistance and Acyclovir or Gancyclovir resistance. Those cells which show the desired phenotype may then be further analyzed by restriction analysis, electrophoresis, Southern analysis, polymerase chain reaction or the like. By identifying fragments which show the presence of the lesion(s) at the target gene site, one can identify cells in which homologous recombination has occurred to inactivate the target gene.

For embryonic stem cells, after mutation, the cells may be plated onto a feeder layer in an appropriate medium, e.g., fetal bovine serum enhanced DMEM. Cells containing the construct may be detected by employing a selective medium and after sufficient time for colonies to grow, colonies may be picked and analyzed for the occurrence of homologous recombination. As described previously, the polymerase chain reaction may be used, with primers within and without the construct sequence but at the target locus. Those colonies which show homologous recombination may then be used for embryo manipulating by blastocyst injection. Blastocysts may be obtained from 4 to 6 week old superovulated females by flushing the uterus 3.5 days after ovulation. The embryonic stem cells may then be trypsinized and the modified cells added to a droplet containing the blastocysts. At least one, usually at least about 10, and up to about 15 of the modified embryonic stem cells may be injected into the blastocoel of the blastocyst. After injection, at least one and not more than about 15 of the blastocysts are returned to each uterine horn of pseudopregnant females. Alternatively, any of the common techniques, i.g. microinjection of the mutated gene, or a fragment thereof, into a one-cell embryo followed by incubation in a foster mother can be used.

The pups will usually be born 16-18 days after introduction of the blastocysts into foster mothers. Chimeric animals will be mated with wild type (wt) mice to create heterozygote transgenics.

With these methods it is possible to obtain transgenic non-human animals, whose one or both allels of a gene encoding a parkin gene are mutated in a way, that a parkin protein with modified, preferred less activity or no active parkin protein is expressed.

"Mutated" means in this content replacements, insertions or deletions of nucleotides or polynucleotide sequences.

In consequence of the mutated parkin gene these animals produce a mutated or truncated parkin protein or no parkin protein. Preferred - if a parkin protein is expressed - the parkin protein expressed by the transgenic animal contains any of the mutations or deletions shown in table 1 and 2, represented by any of the proteins with an amino acid sequence of SEQ ID NO:5, SEQ ID NO:6, SEQ, ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34 or naturally occurring or artificially introduced mutants with a homologous protein sequence or fragments thereof, particularly preferred a parkin protein with a sequence according to SEQ ID NO:21 is expressed.

The expression of one of these proteins or no parkin protein in the transgenic non-human animals causes these animals to display features of a neurodegenerative disease. These features can be manifested in developing physiological, biochemical or molecular biological modifications in e.g. cells, tissues, organs or neuronal structures.

In accordance with standard protocols, cultured eukaryotic cells, either primary cultures or immortalised cell lines, may be transfected, either transiently or stably, with a mutant or fragmented mPark2 allele so that the cultured eukaryotic cell expresses a mutant parkin2 polypeptide.

The present application further refers to cells, typically mammalian cells and preferably mammalian cells of the neural, glial, or astrocytic lineage, that have been transformed or transfected with any DNA sequence according to the present invention, as well as to any cells which have been derived from a transgenic non-human animal, whereby the cells express any of the mutated parkin2 proteins isoforms according to the present invention, preferred any of the isoforms shown in table 1 or 2 or fragments thereof, or they contain a parkin sequence which is mutated in a way that they don't express a parkin protein. The cells derived from the transgenic animals may be cultured as cell-lines or as primary cultures.

Once established, all such cell lines can be grown continuously in culture and may be used for a variety of in vitro experiments to study parkin expression and processing.

The present invention further refers to a method of producing transgenic non-human animals and transformed cells that contain any polynucleotide sequence encoding any mutant mouse parkin2 protein isoform according to the present invention, preferably such as shown in table 1 or 2 or naturally occurring or artificially introduced mutants or fragments thereof.

Preferred the above described polynucleotide sequences, the proteins and amino acid sequences as well as the transgenic animal models and cell lines may be used for any method for analysing the symptoms of neurodegenerative diseases.

Such neurodegenerative diseases encompass among others Parkinson's disease, Alzheimer's disease, Huntigton's disease, amyotrophic lateral sclerosis, Multisystem atrophy, Wilson's disease, Pick's disease, Prion disease, or second causes inducing Parkinson's syndromes like toxins (e.g. Mn, Fe, 6-hydroxydopamine, MPTP (1-methyl-4-phenyl-1,2,3,6-tetrahydropyridine), CO), drugs, brain tumors, head trauma, stroke, vascular irregularities, or metabolic irregularities.

Enclosed to these methods are methods outside of a living body, which are methods of molecular biology like PCR, Southern and Northern blot analysis, construction of DNA or RNA probes, as well as Western blot analysis, preparation of epitopes from the protein or amino acid sequences mentioned in this application, production of monoclonal and polyclonal antibodies. These methods may be used for screening of samples, preferred of biological fluids for either the expression of parkin protein as a method for detecting the presence of the protein, or in a nucleic acid sample or another sample removed from a subject, the presence of the gene for Parkinson's disease comprising identifying a genetic alteration in a gene sequence coding for parkin. Further enclosed are pathobiochemical, immunobiological and neurological as well as histochemical methods carried out after sacrificing the animal for considering the effects of neurodegenerative diseases, particularly Parkinson's disease to the living body. Further methods for locating the presence of genetic alterations associated with Parkinson's disease are provided. These methods may be used outside of a living body to predict the development of the disease prior to onset or for genetic screening.

However, particularly preferred is a method of testing the efficacy of a treatment for a neurodegenerative disease associated with a less active or non-active parkin protein, comprising subjecting any of the created transgenic animals as a model to a putative treatment and determining the efficacy of said treatment.

These testing methods preferably comprise administering an active substance, whose effect can be determined by any of the above described methods, to a transgenic animal according to the present invention.

By the use of the transgenic animals described in the present application it is possible the first time to test in a model system whether an active substance is useful for treating a condition associated with non-active parkin protein and determining a level of the active substance, which causes an effect in treating the disease.

Treatments may carried out as single dose applications, but it is preferred to use the transgenic animals in long-time experiments with multiple dose applications.

The transgenic animals of the present application may be particularly used as model systems for screening for drugs and evaluating drug effectiveness. Additionally, such model systems provide a tool for defining the underlying biochemistry of neurodegenerative diseases, which thereby provides a basis for rational drug design. The models may be used further for studies of behaviour, physiological and molecular biological examinations, pharmacological and toxicological studies and several other applications.

Having detected the genetic mutation in the gene sequence coding for parkin protein in an individual not yet showing overt signs of Parkinson's disease, using any of the methods of the present invention, it may be possible to employ gene therapy, in the form of gene implants, to prevent the development of the disease.

Additional embodiments directed to modulation of the production of variant parkin proteins include methods that employ specific antisense polynucleotides complementary to all or part of a variant parkin sequence according to any of the sequences mentioned in this application, or for some embodiments a wild-type parkin sequence. Such complementary antisense polynucleotides may include nucleotide substitutions, additions, deletions, or transpositions, so long as specific hybridisation to the relevant target sequence is retained as a property of the polynucleotide. Thus, an antisense polynucleotide must preferentially bind to a variant parkin sequence as compared to a wild-type parkin. It is mostly preferred that the antisense polynucleotide reflects the exact nucleotide sequence of the variant allele (or wild-type allele where desired) and not a degenerate sequence.

Complementary antisense polynucleotides include soluble antisense RNA or DNA oligonucleotides which can hybridise specifically to a variant parkin mRNA species and prevent transcription of the mRNA species and/or translation of the encoded polypeptide (Ching et al. (1989) Proc. Natl. Acad. Sci. U.S.A. 86:10006; Broder et al. (1990) Ann. Int. Med. 113:604; Loreau et al. (1990) FEBS Letters 274:53-56); Holcenberg et al. WO91/11535; U.S. Pat. No. 7,530,165 ("New human CRIPTO gene"--publicly available through Derwent Publications Ltd., Rochdale House, 128 Theobalds Road, London, UK); WO91/09865; WO91/04753; WO90/13641; and EP 386563, each of which is incorporated herein by reference). The antisense polynucleotides therefore inhibit production of the variant parkin polypeptides.

Antisense polynucleotides may be produced from a heterologous expression cassette in a transfectant cell or transgenic cell or animal, such as a transgenic neural, glial, or astrocytic cell, preferably where the expression cassette contains a sequence that promotes cell-type specific expression (Wirak et al. loc. cit.). Alternatively, the antisense polynucleotides may comprise soluble oligonucleotides that are administered to the external milieu, either in the culture medium in vitro or in the circulatory system or interstitial fluid in vivo. Soluble antisense polynucleotides present in the external milieu have been shown to gain access to the cytoplasm and inhibit translation of specific mRNA species. In some embodiments the antisense polynucleotides comprise methylphosphonate moieties. For general methods relating to antisense polynucleotides, see Antisense RNA and DNA, (1988), D. A. Melton, Ed., Cold Spring Harbor Laboratory, Cold Spring Harbor, N.Y.).

### Legends to the figures:

Figure 1 shows the alignment of the deduced amino acid sequences of the human and mouse Parkin2 protein (SEQ ID NO: 4).

Underlinded are the conserved ubiquitin like (at the N-terminus) and Ring finger like (at the C-terminus) regions of both proteins.

Figure 2 shows the alignment of the nucleotide sequences of the human and mouse parkin 2 gene. Bold lines represent the exon boundaries identified for the human and mouse sequence.

Figure 3 represents a flow chart of the cloning procedure of the mouse parkin2 gene - exon3 knock-out construct.
Abbreviations:
a) Restriction endonucleases:
   N = NotI, E= Eco RI, B= BamHI, H= HindIII, X= XbaI.
b) Modifications: ()= T4 DNA polymerase treatment in order to remove a restiction site in the resulting plasmid.
c)
   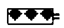 pBluescript KSII (Stratagene) vector sequence
   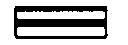 = λ-Fix vector sequence
d) HSV-tk = herpes simples promotor and thymidine kinase gene
e) kb = kilobases

The following examples are provided for illustration and are not intended to limit the invention to the specific example provided.

### Example 1

### Isolation of mouse Parkin2 cDNA clones:

Arrayed mouse brain and mouse kidney cDNA libraries (Biofrontera Pharmaceuticals/ Bio Systems) were screened by PCR under standard conditions using the primers Ex2s:*tcaggttcaactccagctatggc* and Ex2as: *tgcctgcgaaaatcacacgcagc .* The cycle conditions were the following: 3 min. 95°C, (30sec. 95°C, 30sec. 56°C, 1min. 72°C) x 35 cycles.

Single colonies containing the mPark2 genes were verified by colony hybridisation according to the protocol described by Maniatis *et al.* 1989 (see above).

### Construction of the Del Exon3 parkin gene (according to SEQ ID NO: 7)

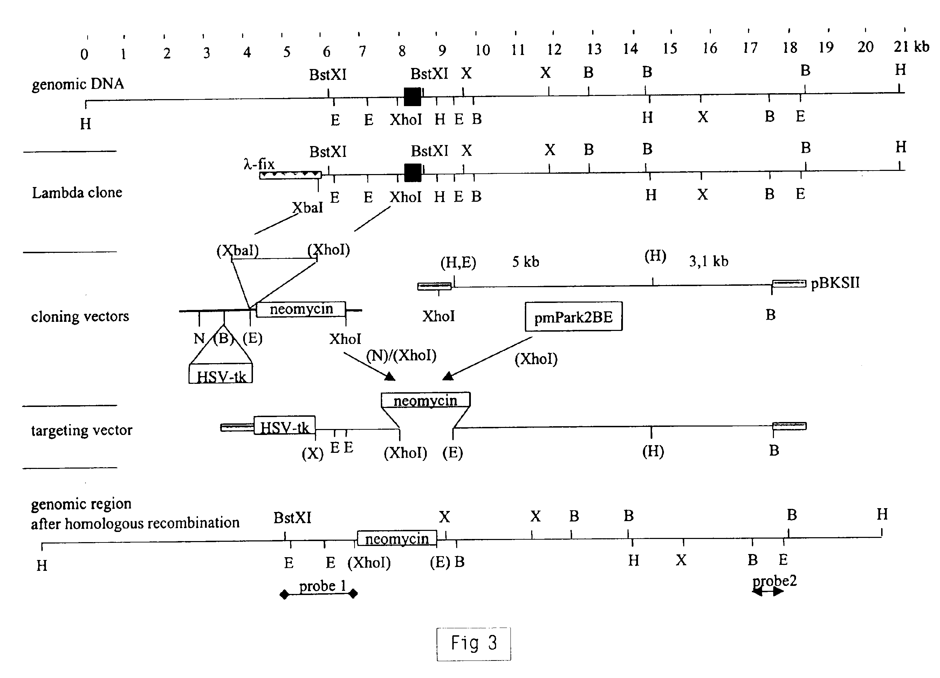

All the further described cloning steps are shown in Figure 3. A genomic lambda ZIP clone (genomic mouse λ-Fix library, Stratagene) containing the exon 3 of the parkin gene was isolated by PCR using exon3 specific primer of the mPark2 gene. A 3.1 kb BamHI/HindIII fragment of the lambda ZIP clone containing genomic DNA 3' end to the exon3 of mPark2 was cloned into the cloning vector pBluescript KS (Stratagene) to obtain the plasmid pmPark2-BH. Secondly, a 5 kb HindIII/EcoRI genomic DNA fragment was inserted into the HindIII site of the pmPark2-BH-clone. The EcoRI and HindIII sites were destroyed by T4 DNA polymerase treatment. As result the plasmid pmPark2-BE- with a 8.1 kb long genomic region to the 3'-end of the exon 3 could be obtained.

A 2.0 kb XbaI/XhoI (the XbaI restriction site is located within the multiple cloning sequence (mcs) of Lambda Fix) genomic DNA fragment containing the genomic region 5' to the exon3 was cloned into the EcoRI site of the pNeoloxp-vector (Giese et al. Science, 1998, 279:870-3 ) after generation of blunt ends by T4 DNA polymerase treatment. The BamHI-site (5'-to the EcoRI-site) of this vector was used subsequently for the insertion of the 2.5 kb HSV-tk-marker gene. Again T4 DNA polymerase was used to generate blunt ends before ligation in order to eliminate the used cloning site. The resulting vector was digested with the restriction enzymes NotI and XhoI to obtain a 6.5 kb fragment containing the HSV-tk, the 2kb XhoI/XbaI genomic region to 5'-end of exon3, and the neo-marker. The vector pmPark2-BE was digested with XhoI to linearise the plasmid. Both the isolated 6.5 kb fragment as well as the linear vector were treated with T4 DNA polymerase prior to ligation to eliminate the used restriction sites.

This plasmid pmPark2del-ex3 was linearised with the restriction enzyme NotI prior to transfection into ES cells.

### Example 2

### Transfection of ES cells:

### Isolation and Freezing of the ES cells:

14 days old embryos were isolated, head and organs were removed from embryos, the remaining tissue was minced, and washed with 1x PBS. 1x trypsin (0,5g/l) / EDTA (0,2g/l) was used for dissolve the tissue by incubating them at 37°C for 5 min. The reaction was stopped by adding 1 vol. EF medium (Embryonic Feeder medium: 1x DMEM, 10% FCS Serum, 2mM Glutamine, all obtained from LIFE Technologies), and cells were dissolved by pipetting several time up and down. The supernatant was centrifuged with 1000 rpm for 5 min. The fibroblasts from one embryo were seeded into a 175 cm² flask with 30 ml medium. The medium was changed after 24 h. When the fibroblasts form a confluent monolayer they were splitted 1:3, and thereafter they were frozen when the cells are confluent again. Cells from 175 cm² flask were frozen into one tube. Therefore first empty tubes are place on ice, freezing medium is added (EF medium + 20% DMSO (Dimethylsulfoxid)), cells with 0.5 ml EF medium are added, mixed, putted in a styrofoam box, which is cooled down in a -80°C freezer, and the next day the tubes are transferred into liquid N₂(l) tank.

### Sub-culturing, inactivation and feeder layer:

The fibroblasts can be cultured on gelatine-coated plastic ware. The cells were splitted carefully 1:3 after 3 days. When feeder layer are needed for ES cell culturing, the fibroblasts should be division-inactivated by mitomycin C. 2 mg mitomycin C are dissolved in 10 ml PBS, which can be stored at -20°C. This stock solution is diluted 1:20 with EF medium for inactivation; the nearly confluent fibroblasts in a 175 cm² flask are incubated in 20-30 ml of medium with mitomycin C for 2 h at 37°C. Mitomycin C is then removed by 2x washing with PBS, and the inactivated fibroblasts are recovered in EF medium for 24 h before they are frozen or used for ES cell culturing after a few days. The cells are stored 37°C until they are used (maximally 10 days;) or they are frozen. For feeder layer, plate cells onto the same area; here the plastic ware has to be coated by gelatine.

### Sub-culturing the ES-cells:

The ES cells were kept for 2-4 passages in culture. The medium is ES medium (1x DMEM, 15% FCS Serum, 2mM Glutamine, 1x nonessential amino acids, 7µl β Mercaptoethanol, with supplement containing LIF (Leukemia Inhibitory Factor, 2.5x10⁵ to 10⁶ U/l),all obtained from LIFE Technologies), and the cells are splitted 1:6 every second day. Cells were refeeded 2 h before passaging.

### Stable Transfection of ES Cells

After digestion of the gene targeting construct the DNA is extracted with phenol/CHCl₃ (24/23) and precipitated with EtOH (wash 2x with 75% EtOH); the rest of EtOH is removed carefully and air dryed for approx. 15 min under steril conditions (laminar flow). The DNA is suspended in H₂O (final conc.: 3 mg/ml). 5x10⁷ cells of a monolayer are treated with 1x trypsin to detach them from the ground of the flask, suspended in 0.8 ml medium and electroporated with DNA (30 µg linear DNA, 800 V, 3 µF, BioRad Gene Pulser). After 20 min at 4°C, cells are diluted with 9.5 ml medium and are plated onto dishes (9 cm diameter). 24 h after electroporation G418 (150-175 mg /ml) is added to start selection. The medium is changed every day; after 7-9 days of selection colonies can be picked.

### Picking colonies and culturing of picked colonies:

24 colonies were picked with Eppendorf tips under an inverted microscope. The colonies were transferred into the wells of a 96-well plate (round bottom), 30 µl lxtrypsin/EDTA are added, and the plates are incubated 10 min at 37°C. Thereafter 100 µl ES-medium are added and the cells are suspended by pipetting up and down 12x with a multichannel pipette. The trypsinized cells are solitarily plated into a 24-well plate. The medium is exchanged every 24 h. 3-4 days after picking the cells are detached from the ground of the plates. Therefore the medium is removed, 60 µl 1xtrypsin/EDTA are added and the plates are incubated for 7 min at 37°C. The treatment is stopped by adding 200 µl medium and the cells are resuspended. 200 µl of the cell suspension is added to 200 ml medium with 20 % DMSO and the cells are frozen as described above.

### Example 3

### DNA isolation and southern blot analysis for control and identification of picked colonies:

To characterize the clones, picked in example 2, DNA is isolated from the cells and examined. Therefore 500 µl medium are added into any well of a picked colony which should still contain 60 µl cell suspension (see example 2). The cells are cultured continuously 3-4 days until confluent for DNA isolation. 500 µl lysis buffer (12 ml 1 M Tris-HCl (pH 8.3); 1.2 ml 0.5 M EDTA; 2.4 ml 10 % SDS; 4.8 ml 5 M NaCl; 1.2 ml 10 mg/ml proteinase K; 98.4 ml H₂O) is added, and it is incubated over night at 55°C. DNA is precipitated by adding 1 vol. 2-propanol and at least 15 min shaking at RT, and transferred with an Eppendorf tip into a 1.5 ml tube with 1 ml 70% EtOH. The tube is centrifuged for 10 min at RT to spin down the DNA. EtOH is removed and pellets are air dried for least one hour. DNA is dissolved afterward in 100 µl TE for over night at 55°C.

### Southern blot analysis:

1/3 of the isolated DNA was used for one digestion. The digestion was carried out for over night at 37°C. Loading buffer was added, and DNAs are separated in an agarose gel for least 6 hours. The gels were incubated in 0.2 N HCl for 15 min at room temperature; after 15 min HCl solution was replaced by 0.4 N NaOH and the gel was incubated therein for 15 min at RT. The DNA was transferred onto nylon membranes (Amersham) over night using 0.4 N NaOH as transfer buffer using a vacuum blot machine (Stratagene). The membranes were neutralized in 2x SSC for 1 min, and air dried for least one hour. After UV-Crosslinking the DNA onto the membrane hybridisation with DNA probes (probes are shown in figure 3) was carried out under standard conditions (QuickHyb from Clontech, 65°C, wash twice with 2x SSC, 0.1 % SDS at 65°C).

### Production of transgenic animals with mutant parkin allele:

10-15 recombinant ES cells are injected into blastocyts. The blastocyts are implanted in pseudopregnant mice. The chimeric spring offs are crossed with wild type mice to obtain heterocygotic recombinant F1 mice. These mice are analysed by southern blot analysis as described above. Transgenic mice are crossed with each other to obtain mice with both alleles modified (homozygote animals).

Descendants of the transgenic animals may be used for breeding with mice strains representing the same or any other genotype, preferred mice strains showing neurological abnormalities, more preferred with strains showing neurodegenerative abnormalities. These other mouse strains may be selected from wild type mice, mice containing knock-ins or knock-outs, mice containing mutants of genes or mice which overexpress any gene product. The most preferred partners for breeding are mice which represent a model for Alzheimer's disease, Huntigton' disease, amyotrophic lateral sclerosis, Multisystem atrophy, Wilson's disease, Pick's disease or Prion disease.

### Use of Transgenic Mice:

The animal can be used to test potential therapeutic agents. The test group of mice is treated with the test compound administered in an appropriate fashion for a set period. At the conclusion of the test period, the animals are assessed behaviourally, biochemically, and histologically for any possible effects of the test compound. The exact protocol depends on the anticipated mechanism of action of the test compound. Compounds that may have utility in treating Parkinson's disease can be identified using this approach.

Such analysis can be carried out in the animal ,in primary tissue cultures of the expressing cells or in immortalised cells derived from those animals.

Mice expressing the truncated parkin2 protein gene or variants of the described one can be used for testing the development of Parkinson's disease during ageing of the animals. Beside the enhanced progression of cell death in substantia nigra area, increased sensitivity to selective neurotoxins like MPTP or 6-hydroxydopamine and enhanced response to dopaminergic precursors like L-dopa may be examined.

## Claims

1. A polynucleotide sequence encoding a mouse parkin2 protein, containing naturally occurring or artificially introduced mutations or deletions, which cause Parkinson's disease in a human if they occur in the according human sequence.

2. The sequence of claim 1, wherein the sequence is genomic DNA, coding for a full-length parkin gene or fragments thereof, cDNA of a full length parkin gene or fragments thereof, or RNA of a full length parkin gene or fragments thereof.

3. The sequence of claim 1 or 2, wherein the sequence is selected from the group, consisting of SEQ ID NO:2, SEQ ID NO:3, SEQ ID NO:7 SEQ ID NO:8, SEQ ID NO:9, SEQ ID NO:10, SEQ ID NO:11, SEQ ID NO:12, SEQ ID NO:13, SEQ ID NO14, SEQ ID NO:15, SEQ ID NO:16, SEQ ID NO:17, SEQ ID NO:18, SEQ ID NO:19, SEQ ID NO:20 or naturally occurring or artificially introduced mutants or fragments thereof.

4. A vector, containing any sequence according to any of claims 1 to 3.

5. A prokaryotic or eukaryotic cell, containing a vector according to claim 4.

6. The cell of claim 5, characterised in that the cell is selected from bacterial or yeast cells, insect cells or mammalian cells as primary cells or immortalised cell lines.

7. A parkin mouse protein with an amino acid sequence of SEQ ID NO:5, SEQ ID NO:6 SEQ, ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34 or naturally occurring or artificially introduced mutants with a homologous protein sequence or fragments thereof.

8. A transgenic non-human animal, whose one or both alleles of a gene encoding a parkin gene are mutated or truncated in a way, that a protein with modified, preferred less activity or no active protein is expressed.

9. The transgenic animal of claim 8, wherein the parkin gene has any mutation or deletion which are known to cause Parkinson's disease in a human if they occur in the according human sequence.

10. The transgenic non-human animal of claim 8 or 9, carrying a mutation or deletion in one or both alleles of a gene encoding a parkin protein, such that expression of said parkin gene produces a mutated or truncated protein or no protein, which causes said animal to display any physiological, biochemical or molecular biological features of a neurodegenerative disease.

11. The transgenic non-human animal of claim 10, carrying a deletion in one or both alleles of any of the exons of the gene encoding the parkin protein.

12. The transgenic non-human animal of any of claims 8 to 11, carrying a DNA sequence according to any of the sequences SEQ ID NO:2, SEQ ID NO:3, SEQ ID NO:7 SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19 or SEQ ID NO:20.

13. A mammalian cell-line transformed or transfected with any sequence according to any of claims 1 to 3 or a vector according to claim 4 or cell lines or primary cultures derived from the transgenic animal of any of claims 8 to 12.

14. A method of producing a transgenic animal according to any of claims 8 to 12 or a cell line according to claim 13.

15. Use of the transgenic non-human animal according to any of claims 8 to 12 or a cell line according to claim 13 as a model for neurodegenerative diseases.

16. A method for analyzing the symptoms of neurodegenerative diseases, either outside of a living body using any of the polynucleotide sequences of any of claims 1 to 4, any of the protein sequences of claim 7, or using any model according to claim 15.

17. A method for testing the efficacy of a treatment for a neurodegenerative disease associated with a less active or non-active parkin protein, comprising subjecting any model of claim 15 to a putative treatment and determining the efficacy of said treatment.

18. The method according to claim 16 or 17, wherein said neurodegenerative disease is selected from the group consisting of Parkinson's disease, Alzheimer's disease, Huntigton's disease, amyotrophic lateral sclerosis, Multisystem atrophy, Wilson's disease, Pick's disease, Prion disease, or second causes inducing Parkinson's syndromes like toxins, drugs, brain tumors, head trauma, stroke, vascular irregularities, or metabolic irregularities.

19. The method of any of claims 17 to 19, wherein said treatment comprises administering an active substance to the model.

20. Use of any model according to claim 15 for testing whether an active substance is useful for treating a condition associated with non-active parkin protein comprising administering said active substance to the transgenic animal of any of claims 8 to 12 or a cell-line of claim 13, and determining a level of the active substance, which causes an effect in treating the disease.

21. Use of the animal according to any of claims 8 to 12 as a model for examination of behaviour during the development of a neurodegenerative disease, or any model according to claim 15 for examination of pathobiochemical, immunobiological, neurological as well as histochemical effects of neurodegenerative diseases, physiological and molecular biological correlation of the disease, for studies of drug effects and for determination of effective drug doses and toxicity.

22. Descendant of the transgenic animal according to any of claims 8 to 12, obtained by breeding with the same or any other genotype.


SEQUENZPROTOKOLL

<110> Firma Biofrontera GmbH

<120> Transgenic animal model for neurodegenerative diseases

<130> 5807EPAlleSequenzen

<140>
<141>

<160> 34

<170> PatentIn Ver. 2.1

<210> 1
<211> 3255
<212> DNA
<213> mouse

<400> 1
ctcagcgagg ggaaggggga ggaggcctgg atgactaaac ctgacagaaa cgctggtggg 60
aggctcgggc gggcgccagt gcccgcgtag gtccttctcg acccgcagcc accacccgcc 120
cggtgaccat gatagtgttt gtcaggttca actccagcta tggcttccca gtggaggtcg 180
attctgacac cagcatcttg cagctcaagg aagtggttgc taagcgacag ggggttccag 240
ctgaccagct gcgtgtgatt tttgccggga aggagcttcc gaatcacctg acggttcaaa 300
actgtgacct ggaacaacag agtattgtac acatagtaca gagaccacgg aggagaagtc 360
atgaaacaaa tgcatctgga ggggacgaac cccagagcac ctcagagggc tccatatggg 420
agtccaggag cttgacacga gtggacctga gcagccatac cctgccggtg gactctgtgg 480
ggctggcggt cattctggac acagacagta agagggattc agaagcagcc agaggtccag 540
ttaaacccac ctacaacagc tttttcatct actgcaaagg cccctgccac aaggtccagc 600
ctggaaagct ccgagttcag tgtggcacct gcaaacaagc aaccctcacc ttggcccagg 660
gcccatcttg ctgggacgat gtcttaattc caaaccggat gagtggtgag tgccagtctc 720
cagactgccc tggaaccaga gctgaatttt tctttaaatg tggagcacac ccaacctcag 780
acaaggacac gtcggtagct ttgaacctga tcaccagcaa caggcgcagc atcccttgca 840
tagcgtgcac agatgtcagg agccctgtcc tggtcttcca gtgtaaccac cgtcacgtga 900
tctgtttgga ctgtttccac ttgtattgtg tcacaagact caacgatcgg cagtttgtcc 960
acgatgctca acttggctac tccctgccgt gtgtagctgg ctgtcccaac tccctgatta 1020
aagagctcca tcacttcagg atccttggag aagagcagta cactaggtac cagcagtatg 1080
gggccgagga atgcgtgctg caaatgggag gtgtgctgtg cccccgtcct ggctgtggag 1140
ctggactgct acctgaacag ggccagagga aagtcacctg cgaagggggc aacggcctgg 1200
gctgcgggtt tgttttctgc cgggactgta aggaagcata ccatgaaggg gattgcgact 1260
cactgctcga accctcagga gccacttctc aggcctacag ggtggacaaa agagccgctg 1320
agcaagctcg ctgggaggag gcctccaagg aaaccatcaa gaagaccacc aagccttgtc 1380
ctcgctgcaa cgtgccaatt gaaaaaaacg gaggatgtat gcacatgaag tgtcctcagc 1440
cccagtgcaa gctggagtgg tgctggaact gtggctgtga gtggaaccga gcctgcatgg 1500
gagatcactg gtttgacgtg tagagagaga tgtcacttgg ccctggacgc acaacctcaa 1560
gggaaactcc gaagattcct accttcctta gccatttctt cttctcgatg catataagca 1620

cataaatgcg cacacacaaa cacaggctgc agattacaga agcagcccct agatcctttc 1680
tagggcaccc acagaaaacc acagcacccg ctggccccag gggaggaggg cactttcagc 1740
ctctggctca ctcgaatgtc agagcttaga tgagggtgca cctttggttt ggattctgta 1800
gaagccatga gtgaggtggg aagtgttttc cagggttgtt gccacgccct gggtaagtaa 1860
cacctctgag gattctcaga agcacacttg agatctgagg aacgctgctc tcatgtagta 1920
atcatctatt cccaaagggc cccctgcagt agtcaaaact atttgtttat ccccccaaat 1980
cctatcttta caaatggtgc tgatgagatt acaacccctc tgtgtactaa tcagcttatc 2040
aaccaagtga gaacctagga aagctaattg gatggcagac tgcttaaatc gcagggagga 2100
ctcagaagcc aaacctactt ccgttcgttt cattatctgc aactttagaa agaaatgatc 2160
tttttttccc cctgaaaaga taacaaagtc tgcaatttgg tttggagtat tcctactgca 2220
gcctggaagt ttagcttcac tgtgaattta acagagaaag tgcctataaa gggggcgttt 2280
ttaagagaca atcccatgat gctgcgccaa tgctaacaac agggtcaaga aacacaatgt 2340
ttatagaagg agcatccctc gaccatctga atgagagtat gcctgacccc ttccaccaca 2400
agtggggaca cctctgcata tctgctccct cctctgctgt taagccccag ggagccccat 2460
ccacccagtg gtcctacaga cagggcaata cacacacacc aagatagcct tcagatcaac 2520
atgcatcaca ctcaagtgtt aatctttcaa ggttttcttt tctttttcct gttttttatt 2580
tgttttgctt ttgctttttt tttttttttt tttggtggtg gtggggctac caaacttgag 2640
gcctagagct aaaaatcata tagaaatgat gttatcttgt ggtgtgagga aaggccagct 2700
ggcctaagtt cacacttttg tcccagtggc cctagactcc acccagccag ctcccaaaat 2760
gaaaagacca cctgtcaagc agcagtcagg agtctgatgt cacccatcac tatttttttt 2820
ccatcattgt gcttgcctct gcctccttcc acacccgtgt gacgtaatcg cattgggaag 2880
ccaggacaat gtttgctgtt ctgctttggg taaagggact ccctgaagct ctgtggctct 2940
ccagtatggt cccttttcct tcctaacaga tgcatatgtt ttcttcagaa tacaatagtg 3000
attcttaaaa taacccaaaa gacaggcatc cacagtgtgt gagcatgaat cacagcctgc 3060
attgtgtgag tgtgaatagt gggataaaag tggatgtcag aagagtggaa atcaaacctc 3120
tgcaaagcaa tctttctctt tctgtgaagt gtattaagaa atacctgaag tctgtgtgtg 3180
tggtggtacc cagactgtca atcaataaag acccagactg tcaatgaaaa aaaaaaaaaa 3240
aaaaaaaaaa aaaaa 3255

<210> 2
<211> 1459
<212> DNA
<213> mouse

<400> 2
ctcacgggga ggaggccttg gatgactaaa cctgacagaa acgctggtgg gaggctcggg 60
cgggcgccag tgcccgcgta ggtccttctc gacccgcagc caccacccgc ccggtgacca 120
tgatagtgtt tgtcaggttc aactccagct atggcttccc agtggaggtc gattctgaca 180
ccagcatctt gcagctcaag gaagtggttg ctaagcgaca gggggttcca gctgaccagc 240
tgcgtgtgat ttttgccggg aaggagcttc cgaatcacct gacggttcaa aactgtgacc 300
tggaacaaca gagtattgta cacatagtac agagaccacg gaggagaagt catgaaacaa 360
atgcatctgg aggggacgaa ccccagagca cctcagaggg ctccatatgg gagtccagga 420
gcttgacacg agtggacctg agcagccata ccctgccggt ggactctgtg gggctggcgg 480
tcattctgga cacagacagt aagagggatt cagaagcagc cagaggtcca gcagttaaac 540
ccacctacaa cagctttttc atctactgca aaggcccctg ccacaaggtc cagcctggaa 600
agctccgagt tcagtgtggc acctgcaaac aagcaaccct caccttggcc cagggcccat 660
cttgctggga cgatgtctta attccaaacc ggatgagtgg tgagtgccag tctccagact 720
gccctggaac cagagctgaa tttttcttta aatgtggagc acacccaacc tcagacaagg 780

acacgtcggt agctttgaac ctgatcacca gcaacaggcg cagcatccct tgcatagcgt 840
gcacagatgt cagtcatctt cctctgtcat ctggtgcctc cgtgtggact cggcctcatc 900
tccactgaac cttgttcttt aggactgtgc aataggtcgt cacctcctac tgagaacaag 960
gcagcttctg gtctcttggt ttccttgctt ccaacggcag cattgactgt acacccttca 1020
gtcctaccaa ccccattacc tggttgattt ctttaccgct tagcttctcc aagatgccta 1080
tttccacaca cagtttcttg tcttccccat ccccccatag gtttatgcgc atgagtaagc 1140
accgcacctc atgagtttgt gcttctgata caagacttcc tgggatcccc gcttgagccc 1200
tagaatcccc tggaactggg ttcagtcacc tatcttcaat agcctctttt aaaagtgagt 1260
tcttggctgg gtgagatggc tcagtgggta agagcacccg actgctcttc cgaagtccag 1320
agttcaaaat cccagcaacc acatggtggc tcacaaccat ccgtaacaag atctgactcc 1380
ctcttctggt gtgtctgaag acagctacag tgtacttaca taaaataata aataaatctt 1440
aaaaaaaaaa aaaaaaaaa 1459

<210> 3
<211> 857
<212> DNA
<213> mouse

<400> 3
ctcagatgac taaacctgac agaaacgctg gtgggaggct cgggcgggcg ccagtgcccg 60
cgtaggtcct tctcgacccg cagccaccac ccgcccggtg accatgatag tgtttgtcag 120
gttcaactcc agctatggct tcccagtgga ggtcgattct gacaccagca tcttgcagct 180
caaggaagtg gttgctaagc gacagggggt tccagctgac cagctgcgtg tgatttttgc 240
cgggaaggag cttccgaatc acctgacggt tcaaaactgt gacctggaac aacagagtat 300
tgtacacata gtacagagac cacggaggag aagtcatgaa acaaatgcat ctggagggga 360
cgaaccccag agcacctcag agggctccat atgggagtcc aggagcttga cacgagtgga 420
cctgagcagc cataccctgc cggtggactc tgtggggctg gcggtcattc tggacacaga 480
cagtaagagg gattcagaag cagccagagg tccagcagtt aaacccacct acaacagctt 540
tttcatctac tgcaaaggcc cctgccacaa ggtccagcct ggaaagctcc gagttcagtg 600
tggcacctgc aaacaagcaa ccctcacctt ggcccagggc ccatcttgct gggacgatgt 660
cttaattcca aaccggatga gtggtgagtg ccagtctcca gactgccctg gaaccagagc 720
tgaatttttc tttaaatgtg gagcacaccc aacctcagac aaggacacgt cggtagcttt 780
gaacctgatc accagcaaca ggcgcagcat cccttgcata gcgtgcacag atgtcaggtt 840
tatgcgcatg agttagc 857

<210> 4
<211> 464
<212> PRT
<213> mouse

<400> 4
Met Ile Val Phe Val Arg Phe Asn Ser Ser Tyr Gly Phe Pro Val Glu
1               5                   10                  15

Val Asp Ser Asp Thr Ser Ile Leu Gln Leu Lys Glu Val Val Ala Lys
            20                  25                  30

Arg Gln Gly Val Pro Ala Asp Gln Leu Arg Val Ile Phe Ala Gly Lys

        35                  40                  45

Glu Leu Pro Asn His Leu Thr Val Gln Asn Cys Asp Leu Glu Gln Gln
    50                  55                  60

Ser Ile Val His Ile Val Gln Arg Pro Arg Arg Arg Ser His Glu Thr
65                  70                  75                  80

Asn Ala Ser Gly Gly Asp Glu Pro Gln Ser Thr Ser Glu Gly Ser Ile
                85                  90                  95

Trp Glu Ser Arg Ser Leu Thr Arg Val Asp Leu Ser Ser His Thr Leu
            100                 105                 110

Pro Val Asp Ser Val Gly Leu Ala Val Ile Leu Asp Thr Asp Ser Lys
        115                 120                 125

Arg Asp Ser Glu Ala Ala Arg Gly Pro Val Lys Pro Thr Tyr Asn Ser
    130                 135                 140

Phe Phe Ile Tyr Cys Lys Gly Pro Cys His Lys Val Gln Pro Gly Lys
145                 150                 155                 160

Leu Arg Val Gln Cys Gly Thr Cys Lys Gln Ala Thr Leu Thr Leu Ala
                165                 170                 175

Gln Gly Pro Ser Cys Trp Asp Asp Val Leu Ile Pro Asn Arg Met Ser
            180                 185                 190

Gly Glu Cys Gln Ser Pro Asp Cys Pro Gly Thr Arg Ala Glu Phe Phe
        195                 200                 205

Phe Lys Cys Gly Ala His Pro Thr Ser Asp Lys Asp Thr Ser Val Ala
    210                 215                 220

Leu Asn Leu Ile Thr Ser Asn Arg Arg Ser Ile Pro Cys Ile Ala Cys
225                 230                 235                 240

Thr Asp Val Arg Ser Pro Val Leu Val Phe Gln Cys Asn His Arg His
                245                 250                 255

Val Ile Cys Leu Asp Cys Phe His Leu Tyr Cys Val Thr Arg Leu Asn
            260                 265                 270

Asp Arg Gln Phe Val His Asp Ala Gln Leu Gly Tyr Ser Leu Pro Cys
        275                 280                 285

Val Ala Gly Cys Pro Asn Ser Leu Ile Lys Glu Leu His His Phe Arg

290                 295                 300

Ile Leu Gly Glu Glu Gln Tyr Thr Arg Tyr Gln Gln Tyr Gly Ala Glu
305                 310                 315                 320

Glu Cys Val Leu Gln Met Gly Gly Val Leu Cys Pro Arg Pro Gly Cys
                325                 330                 335

Gly Ala Gly Leu Leu Pro Glu Gln Gly Gln Arg Lys Val Thr Cys Glu
            340                 345                 350

Gly Gly Asn Gly Leu Gly Cys Gly Phe Val Phe Cys Arg Asp Cys Lys
        355                 360                 365

Glu Ala Tyr His Glu Gly Asp Cys Asp Ser Leu Leu Glu Pro Ser Gly
    370                 375                 380

Ala Thr Ser Gln Ala Tyr Arg Val Asp Lys Arg Ala Ala Glu Gln Ala
385                 390                 395                 400

Arg Trp Glu Glu Ala Ser Lys Glu Thr Ile Lys Lys Thr Thr Lys Pro
                405                 410                 415

Cys Pro Arg Cys Asn Val Pro Ile Glu Lys Asn Gly Gly Cys Met His
            420                 425                 430

Met Lys Cys Pro Gln Pro Gln Cys Lys Leu Glu Trp Cys Trp Asn Cys
        435                 440                 445

Gly Cys Glu Trp Asn Arg Ala Cys Met Gly Asp His Trp Phe Asp Val
    450                 455                 460

<210> 5
<211> 262
<212> PRT
<213> mouse

<400> 5
Met Ile Val Phe Val Arg Phe Asn Ser Ser Tyr Gly Phe Pro Val Glu
  1               5                  10                  15

Val Asp Ser Asp Thr Ser Ile Leu Gln Leu Lys Glu Val Val Ala Lys
             20                  25                  30

Arg Gln Gly Val Pro Ala Asp Gln Leu Arg Val Ile Phe Ala Gly Lys
        35                  40                  45

Glu Leu Pro Asn His Leu Thr Val Gln Asn Cys Asp Leu Glu Gln Gln
    50                  55                  60

Ser Ile Val His Ile Val Gln Arg Pro Arg Arg Arg Ser His Glu Thr
65                  70                  75                  80

Asn Ala Ser Gly Gly Asp Glu Pro Gln Ser Thr Ser Glu Gly Ser Ile
                85                  90                  95

Trp Glu Ser Arg Ser Leu Thr Arg Val Asp Leu Ser Ser His Thr Leu
            100                 105                 110

Pro Val Asp Ser Val Gly Leu Ala Val Ile Leu Asp Thr Asp Ser Lys
        115                 120                 125

Arg Asp Ser Glu Ala Ala Arg Gly Pro Ala Val Lys Pro Thr Tyr Asn
    130                 135                 140

Ser Phe Phe Ile Tyr Cys Lys Gly Pro Cys His Lys Val Gln Pro Gly
145                 150                 155                 160

Lys Leu Arg Val Gln Cys Gly Thr Cys Lys Gln Ala Thr Leu Thr Leu
                165                 170                 175

Ala Gln Gly Pro Ser Cys Trp Asp Asp Val Leu Ile Pro Asn Arg Met
            180                 185                 190

Ser Gly Glu Cys Gln Ser Pro Asp Cys Pro Gly Thr Arg Ala Glu Phe
        195                 200                 205

Phe Phe Lys Cys Gly Ala His Pro Thr Ser Asp Lys Asp Thr Ser Val
    210                 215                 220

Ala Leu Asn Leu Ile Thr Ser Asn Arg Arg Ser Ile Pro Cys Ile Ala
225                 230                 235                 240

Cys Thr Asp Val Ser His Leu Pro Leu Ser Ser Gly Ala Ser Val Trp
                245                 250                 255

Thr Arg Pro His Leu His
            260

<210> 6
<211> 250

<212> PRT
<213> mouse

<400> 6
Met Ile Val Phe Val Arg Phe Asn Ser Ser Tyr Gly Phe Pro Val Glu
1               5                   10                  15

Val Asp Ser Asp Thr Ser Ile Leu Gln Leu Lys Glu Val Val Ala Lys
            20                  25                  30

Arg Gln Gly Val Pro Ala Asp Gln Leu Arg Val Ile Phe Ala Gly Lys
        35                  40                  45

Glu Leu Pro Asn His Leu Thr Val Gln Asn Cys Asp Leu Glu Gln Gln
    50                  55                  60

Ser Ile Val His Ile Val Gln Arg Pro Arg Arg Arg Ser His Glu Thr
65                  70                  75                  80

Asn Ala Ser Gly Gly Asp Glu Pro Gln Ser Thr Ser Glu Gly Ser Ile
                85                  90                  95

Trp Glu Ser Arg Ser Leu Thr Arg Val Asp Leu Ser Ser His Thr Leu
            100                 105                 110

Pro Val Asp Ser Val Gly Leu Ala Val Ile Leu Asp Thr Asp Ser Lys
        115                 120                 125

Arg Asp Ser Glu Ala Ala Arg Gly Pro Ala Val Lys Pro Thr Tyr Asn
    130                 135                 140

Ser Phe Phe Ile Tyr Cys Lys Gly Pro Cys His Lys Val Gln Pro Gly
145                 150                 155                 160

Lys Leu Arg Val Gln Cys Gly Thr Cys Lys Gln Ala Thr Leu Thr Leu
                165                 170                 175

Ala Gln Gly Pro Ser Cys Trp Asp Asp Val Leu Ile Pro Asn Arg Met
            180                 185                 190

Ser Gly Glu Cys Gln Ser Pro Asp Cys Pro Gly Thr Arg Ala Glu Phe
        195                 200                 205

Phe Phe Lys Cys Gly Ala His Pro Thr Ser Asp Lys Asp Thr Ser Val
    210                 215                 220

Ala Leu Asn Leu Ile Thr Ser Asn Arg Arg Ser Ile Pro Cys Ile Ala
225                 230                 235                 240

Cys Thr Asp Val Arg Phe Met Arg Met Ser
                245                 250

<210> 7
<211> 3014
<212> DNA
<213> mouse

<400> 7
ctcagcgagg ggaaggggga ggaggcctgg atgactaaac ctgacagaaa cgctggtggg 60
aggctcgggc gggcgccagt gcccgcgtag gtccttctcg acccgcagcc accacccgcc 120
cggtgaccat gatagtgttt gtcaggttca actccagcta tggcttccca gtggaggtcg 180
attctgacac cagcatcttg cagctcaagg aagtggttgc taagcgacag ggggttccag 240
ctgaccagct gcgtgtgatt tttgccggga aggagcttcc gaatcacctg acggttcaat 300
taaacccacc tacaacagct ttttcatcta ctgcaaaggc ccctgccaca aggtccagcc 360
tggaaagctc cgagttcagt gtggcacctg caaacaagca accctcacct tggcccaggg 420
cccatcttgc tgggacgatg tcttaattcc aaaccggatg agtggtgagt gccagtctcc 480
agactgccct ggaaccagag ctgaattttt ctttaaatgt ggagcacacc caacctcaga 540
caaggacacg tcggtagctt tgaacctgat caccagcaac aggcgcagca tcccttgcat 600
agcgtgcaca gatgtcagga gccctgtcct ggtcttccag tgtaaccacc gtcacgtgat 660
ctgtttggac tgtttccact tgtattgtgt cacaagactc aacgatcggc agtttgtcca 720
cgatgctcaa cttggctact ccctgccgtg tgtagctggc tgtcccaact ccctgattaa 780
agagctccat cacttcagga tccttggaga agagcagtac actaggtacc agcagtatgg 840
ggccgaggaa tgcgtgctgc aaatgggagg tgtgctgtgc ccccgtcctg gctgtggagc 900
tggactgcta cctgaacagg gccagaggaa agtcacctgc gaagggggca acggcctggg 960
ctgcgggttt gttttctgcc gggactgtaa ggaagcatac catgaagggg attgcgactc 1020
actgctcgaa ccctcaggag ccacttctca ggcctacagg gtggacaaaa gagccgctga 1080
gcaagctcgc tgggaggagg cctccaagga aaccatcaag aagaccacca agccttgtcc 1140
tcgctgcaac gtgccaattg aaaaaaacgg aggatgtatg cacatgaagt gtcctcagcc 1200
ccagtgcaag ctggagtggt gctggaactg tggctgtgag tggaaccgag cctgcatggg 1260
agatcactgg tttgacgtgt agagagagat gtcacttggc cctggacgca caacctcaag 1320
ggaaactccg aagattccta ccttccttag ccatttcttc ttctcgatgc atataagcac 1380
ataaatgcgc acacacaaac acaggctgca gattacagaa gcagccccta gatcctttct 1440
agggcaccca cagaaaacca cagcacccgc tggccccagg gggaggaggc actttcagcc 1500
tctggctcac tcgaatgtca gagcttagat gagggtgcac ctttggtttg gattctgtag 1560
aagccatgag tgaggtggga agtgttttcc agggttgttg ccacgccctg ggtaagtaac 1620
acctctgagg attctcagaa gcacacttga gatctgagga acgctgctct catgtagtaa 1680
tcatctattc ccaaagggcc ccctgcagta gtcaaaacta tttgtttatc cccccaaatc 1740
ctatctttac aaatggtgct gatgagatta caacccctct gtgtactaat cagcttatca 1800
accaagtgag aacctaggaa agctaattgg atggcagact gcttaaatcg cagggaggac 1860
tcagaagcca aacctacttc cgttcgtttc attatctgca actttagaaa gaaatgatct 1920
ttttttcccc ctgaaaagat aacaaagtct gcaatttggt ttggagtatt cctactgcag 1980
cctggaagtt tagcttcact gtgaatttaa cagagaaagt gcctataaag ggggcgtttt 2040
taagagacaa tcccatgatg ctgcgccaat gctaacaaca gggtcaagaa acacaatgtt 2100
tatagaagga gcatccctcg accatctgaa tgagagtatg cctgacccct tccaccacaa 2160
gtggggacac ctctgcatat ctgctccctc ctctgctgtt aagccccagg gagccccatc 2220

cacccagtgg tcctacagac agggcaatac acacacacca agatagcctt cagatcaaca 2280
tgcatcacac tcaagtgtta atctttcaag gttttctttt ctttttcctg ttttttattt 2340
gttttgcttt tgcttttttt tttttttttt ttggtggtgg tggggctacc aaacttgagg 2400
cctagagcta aaaatcatat agaaatgatg ttatcttgtg gtgtgaggaa aggccagctg 2460
gcctaagttc acacttttgt cccagtggcc ctagactcca cccagccagc tcccaaaatg 2520
aaaagaccac ctgtcaagca gcagtcagga gtctgatgtc acccatcact attttttttc 2580
catcattgtg cttgcctctg cctccttcca cacccgtgtg acgtaatcgc attgggaagc 2640
caggacaatg tttgctgttc tgctttgggt aaagggactc cctgaagctc tgtggctctc 2700
cagtatggtc ccttttcctt cctaacagat gcatatgttt tcttcagaat acaatagtga 2760
ttcttaaaat aacccaaaag acaggcatcc acagtgtgtg agcatgaatc acagcctgca 2820
ttgtgtgagt gtgaatagtg ggataaaagt ggatgtcaga agagtggaaa tcaaacctct 2880
gcaaagcaat ctttctcttt ctgtgaagtg tattaagaaa tacctgaagt ctgtgtgtgt 2940
ggtggtaccc agactgtcaa tcaataaaga cccagactgt caatgaaaaa aaaaaaaaaa 3000
aaaaaaaaaa aaaa 3014

<210> 8
<211> 2895
<212> DNA
<213> mouse

<400> 8
ctcagcgagg ggaaggggga ggaggcctgg atgactaaac ctgacagaaa cgctggtggg 60
aggctcgggc gggcgccagt gcccgcgtag gtccttctcg acccgcagcc accacccgcc 120
cggtgaccat gatagtgttt gtcaggttca actccagcta tggcttccca gtggaggtcg 180
attctgacac cagcatcttg cagctcaagg aagtggttgc taagcgacag ggggttccag 240
ctgaccagct gcgtgtgatt tttgccggga aggagcttcc gaatcacctg acggttcaag 300
gcccatcttg ctgggacgat gtcttaattc caaaccggat gagtggtgag tgccagtctc 360
cagactgccc tggaaccaga gctgaatttt tctttaaatg tggagcacac ccaacctcag 420
acaaggacac gtcggtagct ttgaacctga tcaccagcaa caggcgcagc atcccttgca 480
tagcgtgcac agatgtcagg agccctgtcc tggtcttcca gtgtaaccac cgtcacgtga 540
tctgtttgga ctgtttccac ttgtattgtg tcacaagact caacgatcgg cagtttgtcc 600
acgatgctca acttggctac tccctgccgt gtgtagctgg ctgtcccaac tccctgatta 660
aagagctcca tcacttcagg atccttggag aagagcagta cactaggtac cagcagtatg 720
gggccgagga atgcgtgctg caaatgggag gtgtgctgtg cccccgtcct ggctgtggag 780
ctggactgct acctgaacag ggccagagga aagtcacctg cgaagggggc aacggcctgg 840
gctgcgggtt tgttttctgc cgggactgta aggaagcata ccatgaaggg gattgcgact 900
cactgctcga accctcagga gccacttctc aggcctacag ggtggacaaa agagccgctg 960
agcaagctcg ctgggaggag gcctccaagg aaaccatcaa gaagaccacc aagccttgtc 1020
ctcgctgcaa cgtgccaatt gaaaaaaacg gaggatgtat gcacatgaag tgtcctcagc 1080
cccagtgcaa gctggagtgg tgctggaact gtggctgtga gtggaaccga gcctgcatgg 1140
gagatcactg gtttgacgtg tagagagaga tgtcacttgg ccctggacgc acaacctcaa 1200
gggaaactcc gaagattcct accttcctta gccatttctt cttctcgatg catataagca 1260
cataaatgcg cacacacaaa cacaggctgc agattacaga agcagcccct agatcctttc 1320
tagggcaccc acagaaaacc acagcacccg ctggccccag ggggaggagg cactttcagc 1380
ctctggctca ctcgaatgtc agagcttaga tgagggtgca cctttggttt ggattctgta 1440
gaagccatga gtgaggtggg aagtgttttc cagggttgtt gccacgccct gggtaagtaa 1500
cacctctgag gattctcaga agcacacttg agatctgagg aacgctgctc tcatgtagta 1560
atcatctatt cccaaagggc cccctgcagt agtcaaaact atttgtttat ccccccaaat 1620

cctatcttta caaatggtgc tgatgagatt acaacccctc tgtgtactaa tcagcttatc 1680
aaccaagtga gaacctagga aagctaattg gatggcagac tgcttaaatc gcagggagga 1740
ctcagaagcc aaacctactt ccgttcgttt cattatctgc aactttagaa agaaatgatc 1800
tttttttccc cctgaaaaga taacaaagtc tgcaatttgg tttggagtat tcctactgca 1860
gcctggaagt ttagcttcac tgtgaattta acagagaaag tgcctataaa gggggcgttt 1920
ttaagagaca atcccatgat gctgcgccaa tgctaacaac agggtcaaga aacacaatgt 1980
ttatagaagg agcatccctc gaccatctga atgagagtat gcctgacccc ttccaccaca 2040
agtggggaca cctctgcata tctgctccct cctctgctgt taagccccag ggagccccat 2100
ccacccagtg gtcctacaga cagggcaata cacacacacc aagatagcct tcagatcaac 2160
atgcatcaca ctcaagtgtt aatctttcaa ggtttctttt tctttttcct gtttttttatt 2220
tgttttgctt ttgctttttt tttttttttt tttggtggtg gtggggctac caaacttgag 2280
gcctagagct aaaaatcata tagaaatgat gttatcttgt ggtgtgagga aaggccagct 2340
ggcctaagtt cacacttttg tcccagtggc cctagactcc acccagccag ctcccaaaat 2400
gaaaagacca cctgtcaagc agcagtcagg agtctgatgt cacccatcac tatttttttt 2460
ccatcattgt gcttgcctct gcctccttcc acacccgtgt gacgtaatcg cattgggaag 2520
ccaggacaat gtttgctgtt ctgctttggg taaagggact ccctgaagct ctgtggctct 2580
ccagtatggt cccttttcct tcctaacaga tgcatatgtt ttcttcagaa tacaatagtg 2640
attcttaaaa taacccaaaa gacaggcatc cacagtgtgt gagcatgaat cacagcctgc 2700
attgtgtgag tgtgaatagt gggataaaag tggatgtcag aagagtggaa atcaaacctc 2760
tgcaaagcaa tctttctctt tctgtgaagt gtattaagaa atacctgaag tctgtgtgtg 2820
tggtggtacc cagactgtca atcaataaag acccagactg tcaatgaaaa aaaaaaaaaa 2880
aaaaaaaaaa aaaaa 2895

<210> 9
<211> 2558
<212> DNA
<213> mouse

<400> 9
ctcagcgagg ggaaggggga ggaggcctgg atgactaaac ctgacagaaa cgctggtggg 60
aggctcgggc gggcgccagt gcccgcgtag gtccttctcg acccgcagcc accacccgcc 120
cggtgaccat gatagtgttt gtcaggttca actccagcta tggcttccca gtggaggtcg 180
attctgacac cagcatcttg cagctcaagg aagtggttgc taagcgacag ggggttccag 240
ctgaccagct gcgtgtgatt tttgccggga aggagcttcc gaatcacctg acggttcaac 300
tggctgtccc aactccctga ttaaagagct ccatcacttc aggatccttg gagaagagca 360
gtacactagg taccagcagt atggggccga ggaatgcgtg ctgcaaatgg gaggtgtgct 420
gtgcccccgt cctggctgtg gagctggact gctacctgaa cagggccaga ggaaagtcac 480
ctgcgaaggg ggcaacggcc tgggctgcgg gtttgttttc tgccgggact gtaaggaagc 540
ataccatgaa ggggattgcg actcactgct cgaaccctca ggagccactt ctcaggccta 600
cagggtggac aaaagagccg ctgagcaagc tcgctgggag gaggcctcca aggaaaccat 660
caagaagacc accaagcctt gtcctcgctg caacgtgcca attgaaaaaa acggaggatg 720
tatgcacatg aagtgtcctc agccccagtg caagctggag tggtgctgga actgtggctg 780
tgagtggaac cgagcctgca tgggagatca ctggtttgac gtgtagagag agatgtcact 840
tggccctgga cgcacaacct caagggaaac tccgaagatt cctaccttcc ttagccattt 900
cttcttctcg atgcatataa gcacataaat gcgcacacac aaacacaggc tgcagattac 960
agaagcagcc cctagatcct ttctagggca cccacagaaa accacagcac ccgctggccc 1020
cagggggagg aggcactttc agcctctggc tcactcgaat gtcagagctt agatgagggt 1080
gcacctttgg tttggattct gtagaagcca tgagtgaggt gggaagtgtt ttccagggtt 1140

gttgccacgc cctgggtaag taacacctct gaggattctc agaagcacac ttgagatctg 1200
aggaacgctg ctctcatgta gtaatcatct attcccaaag ggccccctgc agtagtcaaa 1260
actatttgtt tatcccccca aatcctatct ttacaaatgg tgctgatgag attacaaccc 1320
ctctgtgtac taatcagctt atcaaccaag tgagaaccta ggaaagctaa ttggatggca 1380
gactgcttaa atcgcaggga ggactcagaa gccaaaccta cttccgttcg tttcattatc 1440
tgcaacttta gaaagaaatg atcttttttt ccccctgaaa agataacaaa gtctgcaatt 1500
tggtttggag tattcctact gcagcctgga agtttagctt cactgtgaat ttaacagaga 1560
aagtgcctat aaagggggcg tttttaagag acaatcccat gatgctgcgc caatgctaac 1620
aacagggtca agaaacacaa tgtttataga aggagcatcc ctcgaccatc tgaatgagag 1680
tatgcctgac cccttccacc acaagtgggg acacctctgc atatctgctc cctcctctgc 1740
tgttaagccc cagggagccc catccaccca gtggtcctac agacagggca atacacacac 1800
accaagatag ccttcagatc aacatgcatc acactcaagt gttaatcttt caaggttttc 1860
ttttcttttt cctgtttttt atttgttttg cttttgcttt tttttttttt ttttttggtg 1920
gtggtggggc taccaaactt gaggcctaga gctaaaaatc atatagaaat gatgttatct 1980
tgtggtgtga ggaaaggcca gctggcctaa gttcacactt ttgtcccagt ggccctagac 2040
tccacccagc cagctcccaa aatgaaaaga ccacctgtca agcagcagtc aggagtctga 2100
tgtcacccat cactattttt tttccatcat tgtgcttgcc tctgcctcct tccacacccg 2160
tgtgacgtaa tcgcattggg aagccaggac aatgtttgct gttctgcttt gggtaaaggg 2220
actccctgaa gctctgtggc tctccagtat ggtccctttt ccttcctaac agatgcatat 2280
gttttcttca gaatacaata gtgattctta aaataaccca aaagacaggc atccacagtg 2340
tgtgagcatg aatcacagcc tgcattgtgt gagtgtgaat agtgggataa aagtggatgt 2400
cagaagagtg gaaatcaaac ctctgcaaag caatctttct ctttctgtga agtgtattaa 2460
gaaatacctg aagtctgtgt gtgtggtggt acccagactg tcaatcaata aagacccaga 2520
ctgtcaatga aaaaaaaaaa aaaaaaaaaa aaaaaaa 2558

<210> 10
<211> 3136
<212> DNA
<213> mouse

<400> 10
ctcagcgagg ggaaggggga ggaggcctgg atgactaaac ctgacagaaa cgctggtggg 60
aggctcgggc gggcgccagt gcccgcgtag gtccttctcg acccgcagcc accacccgcc 120
cggtgaccat gatagtgttt gtcaggttca actccagcta tggcttccca gtggaggtcg 180
attctgacac cagcatcttg cagctcaagg aagtggttgc taagcgacag ggggttccag 240
ctgaccagct gcgtgtgatt tttgccggga aggagcttcc gaatcacctg acggttcaaa 300
actgtgacct ggaacaacag agtattgtac acatagtaca gagaccacgg aggagaagtc 360
atgaaacaaa tgcatctgga ggggacgaac cccagagcac ctcagagggc tccatatggg 420
agtccaggag cttgacacga gtggacctga gcagccatac cctgccggtg gactctgtgg 480
ggctggcggt cattctggac acagacagta agagggattc agaagcagcc agaggtccag 540
ggcccatctt gctgggacga tgtcttaatt ccaaaccgga tgagtggtga gtgccagtct 600
ccagactgcc ctggaaccag agctgaattt ttctttaaat gtggagcaca cccaacctca 660
gacaaggaca cgtcggtagc tttgaacctg atcaccagca acaggcgcag catcccttgc 720
atagcgtgca cagatgtcag gagccctgtc ctggtcttcc agtgtaacca ccgtcacgtg 780
atctgtttgg actgtttcca cttgtattgt gtcacaagac tcaacgatcg gcagtttgtc 840
cacgatgctc aacttggcta ctccctgccg tgtgtagctg gctgtcccaa ctccctgatt 900
aaagagctcc atcacttcag gatccttgga gaagagcagt acactaggta ccagcagtat 960
ggggccgagg aatgcgtgct gcaaatggga ggtgtgctgt gcccccgtcc tggctgtgga 1020

gctggactgc tacctgaaca gggccagagg aaagtcacct gcgaaggggg caacggcctg 1080
ggctgcgggt ttgttttctg ccgggactgt aaggaagcat accatgaagg ggattgcgac 1140
tcactgctcg aaccctcagg agccacttct caggcctaca gggtggacaa aagagccgct 1200
gagcaagctc gctgggagga ggcctccaag gaaaccatca agaagaccac caagccttgt 1260
cctcgctgca acgtgccaat tgaaaaaaac ggaggatgta tgcacatgaa gtgtcctcag 1320
ccccagtgca agctggagtg gtgctggaac tgtggctgtg agtggaaccg agcctgcatg 1380
ggagatcact ggtttgacgt gtagagagag atgtcacttg gccctggacg cacaacctca 1440
agggaaactc cgaagattcc taccttcctt agccatttct tcttctcgat gcatataagc 1500
acataaatgc gcacacacaa acacaggctg cagattacag aagcagcccc tagatccttt 1560
ctagggcacc cacagaaaac cacagcaccc gctggcccca gggggaggag gcactttcag 1620
cctctggctc actcgaatgt cagagcttag atgagggtgc acctttggtt tggattctgt 1680
agaagccatg agtgaggtgg gaagtgtttt ccagggttgt tgccacgccc tgggtaagta 1740
acacctctga ggattctcag aagcacactt gagatctgag gaacgctgct ctcatgtagt 1800
aatcatctat tcccaaaggg ccccctgcag tagtcaaaac tatttgttta tccccccaaa 1860
tcctatcttt acaaatggtg ctgatgagat tacaacccct ctgtgtacta atcagcttat 1920
caaccaagtg agaacctagg aaagctaatt ggatggcaga ctgcttaaat cgcagggagg 1980
actcagaagc caaacctact tccgttcgtt tcattatctg caactttaga aagaaatgat 2040
ctttttttcc ccctgaaaag ataacaaagt ctgcaatttg gtttggagta ttcctactgc 2100
agcctggaag tttagcttca ctgtgaattt aacagagaaa gtgcctataa agggggcgtt 2160
tttaagagac aatcccatga tgctgcgcca atgctaacaa cagggtcaag aaacacaatg 2220
tttatagaag gagcatccct cgaccatctg aatgagagta tgcctgaccc cttccaccac 2280
aagtggggac acctctgcat atctgctccc tcctctgctg ttaagcccca gggagcccca 2340
tccacccagt ggtcctacag acagggcaat acacacacac caagatagcc ttcagatcaa 2400
catgcatcac actcaagtgt taatctttca aggttttctt ttctttttcc tgttttttat 2460
ttgttttgct tttgcttttt tttttttttt ttttggtggt ggtggggcta ccaaacttga 2520
ggcctagagc taaaaatcat atagaaatga tgttatcttg tggtgtgagg aaaggccagc 2580
tggcctaagt tcacactttt gtcccagtgg ccctagactc cacccagcca gctcccaaaa 2640
tgaaaagacc acctgtcaag cagcagtcag gagtctgatg tcacccatca ctattttttt 2700
tccatcattg tgcttgcctc tgcctccttc cacacccgtg tgacgtaatc gcattgggaa 2760
gccaggacaa tgtttgctgt tctgctttgg gtaaagggac tccctgaagc tctgtggctc 2820
tccagtatgg tcccttttcc ttcctaacag atgcatatgt tttcttcaga atacaatagt 2880
gattcttaaa ataacccaaa agacaggcat ccacagtgtg tgagcatgaa tcacagcctg 2940
cattgtgtga gtgtgaatag tgggataaaa gtggatgtca gaagagtgga aatcaaacct 3000
ctgcaaagca atctttctct ttctgtgaag tgtattaaga aatacctgaa gtctgtgtgt 3060
gtggtggtac ccagactgtc aatcaataaa gacccagact gtcaatgaaa aaaaaaaaaa 3120
aaaaaaaaaa aaaaaa 3136

<210> 11
<211> 3170
<212> DNA
<213> mouse

<400> 11
ctcagcgagg ggaaggggga ggaggcctgg atgactaaac ctgacagaaa cgctggtggg 60
aggctcgggc gggcgccagt gcccgcgtag gtccttctcg acccgcagcc accacccgcc 120
cggtgaccat gatagtgttt gtcaggttca actccagcta tggcttccca gtggaggtcg 180
attctgacac cagcatcttg cagctcaagg aagtggttgc taagcgacag ggggttccag 240
ctgaccagct gcgtgtgatt tttgccggga aggagcttcc gaatcacctg acggttcaaa 300

actgtgacct ggaacaacag agtattgtac acatagtaca gagaccacgg aggagaagtc 360
atgaaacaaa tgcatctgga ggggacgaac cccagagcac ctcagagggc tccatatggg 420
agtccaggag cttgacacga gtggacctga gcagccatac cctgccggtg gactctgtgg 480
ggctggcggt cattctggac acagacagta agagggattc agaagcagcc agaggtccag 540
ttaaacccac ctacaacagc tttttcatct actgcaaagg cccctgccac aaggtccagc 600
ctggaaagct ccgagttcag tgtggcacct gcaaacaagc aaccctcacc ttggcccaga 660
atttttcttt aaatgtggag cacacccaac ctcagacaag gacacgtcgg tagctttgaa 720
cctgatcacc agcaacaggc gcagcatccc ttgcatagcg tgcacagatg tcaggagccc 780
tgtcctggtc ttccagtgta accaccgtca cgtgatctgt ttggactgtt tccacttgta 840
ttgtgtcaca agactcaacg atcggcagtt tgtccacgat gctcaacttg gctactccct 900
gccgtgtgta gctggctgtc ccaactccct gattaaagag ctccatcact tcaggatcct 960
tggagaagag cagtacacta ggtaccagca gtatggggcc gaggaatgcg tgctgcaaat 1020
gggaggtgtg ctgtgccccc gtcctggctg tggagctgga ctgctacctg aacagggcca 1080
gaggaaagtc acctgcgaag ggggcaacgg cctgggctgc gggtttgttt tctgccggga 1140
ctgtaaggaa gcataccatg aaggggattg cgactcactg ctcgaaccct caggagccac 1200
ttctcaggcc tacagggtgg acaaaagagc cgctgagcaa gctcgctggg aggaggcctc 1260
caaggaaacc atcaagaaga ccaccaagcc ttgtcctcgc tgcaacgtgc caattgaaaa 1320
aaacggagga tgtatgcaca tgaagtgtcc tcagccccag tgcaagctgg agtggtgctg 1380
gaactgtggc tgtgagtgga accgagcctg catgggagat cactggtttg acgtgtagag 1440
agagatgtca cttggccctg gacgcacaac ctcaagggaa actccgaaga ttcctacctt 1500
ccttagccat ttcttcttct cgatgcatat aagcacataa atgcgcacac acaaacacag 1560
gctgcagatt acagaagcag cccctagatc ctttctaggg cacccacaga aaaccacagc 1620
acccgctggc cccaggggga ggaggcactt tcagcctctg gctcactcga atgtcagagc 1680
ttagatgagg gtgcaccttt ggtttggatt ctgtagaagc catgagtgag gtgggaagtg 1740
ttttccaggg ttgttgccac gccctgggta agtaacacct ctgaggattc tcagaagcac 1800
acttgagatc tgaggaacgc tgctctcatg tagtaatcat ctattcccaa agggccccct 1860
gcagtagtca aaactatttg tttatccccc caaatcctat ctttacaaat ggtgctgatg 1920
agattacaac ccctctgtgt actaatcagc ttatcaacca agtgagaacc taggaaagct 1980
aattggatgg cagactgctt aaatcgcagg gaggactcag aagccaaacc tacttccgtt 2040
cgtttcatta tctgcaactt tagaaagaaa tgatcttttt ttccccctga aaagataaca 2100
aagtctgcaa tttggtttgg agtattccta ctgcagcctg gaagtttagc ttcactgtga 2160
atttaacaga gaaagtgcct ataaaggggg cgtttttaag agacaatccc atgatgctgc 2220
gccaatgcta acaacagggt caagaaacac aatgtttata gaaggagcat ccctcgacca 2280
tctgaatgag agtatgcctg accccttcca ccacaagtgg ggacacctct gcatatctgc 2340
tccctcctct gctgttaagc cccagggagc cccatccacc cagtggtcct acagacaggg 2400
caatacacac acaccaagat agccttcaga tcaacatgca tcacactcaa gtgttaatct 2460
ttcaaggttt tcttttcttt ttcctgtttt ttatttgttt tgcttttgct tttttttttt 2520
ttttttttgg tggtggtggg gctaccaaac ttgaggccta gagctaaaaa tcatatagaa 2580
atgatgttat cttgtggtgt gaggaaaggc cagctggcct aagttcacac ttttgtccca 2640
gtggccctag actccaccca gccagctccc aaaatgaaaa gaccacctgt caagcagcag 2700
tcaggagtct gatgtcaccc atcactattt tttttccatc attgtgcttg cctctgcctc 2760
cttccacacc cgtgtgacgt aatcgcattg ggaagccagg acaatgtttg ctgttctgct 2820
ttgggtaaag ggactccctg aagctctgtg gctctccagt atggtccctt ttccttccta 2880
acagatgcat atgttttctt cagaatacaa tagtgattct taaaataacc caaaagacag 2940
gcatccacag tgtgtgagca tgaatcacag cctgcattgt gtgagtgtga atagtgggat 3000
aaaagtggat gtcagaagag tggaaatcaa acctctgcaa agcaatcttt ctctttctgt 3060
gaagtgtatt aagaaatacc tgaagtctgt gtgtgtggtg gtacccagac tgtcaatcaa 3120
taaagaccca gactgtcaat gaaaaaaaaa aaaaaaaaaa aaaaaaaaaa 3170

<210> 12
<211> 2918
<212> DNA
<213> mouse

<400> 12
ctcagcgagg ggaaggggga ggaggcctgg atgactaaac ctgacagaaa cgctggtggg 60
aggctcgggc gggcgccagt gcccgcgtag gtccttctcg acccgcagcc accacccgcc 120
cggtgaccat gatagtgttt gtcaggttca actccagcta tggcttccca gtggaggtcg 180
attctgacac cagcatcttg cagctcaagg aagtggttgc taagcgacag ggggttccag 240
ctgaccagct gcgtgtgatt tttgccggga aggagcttcc gaatcacctg acggttcaaa 300
actgtgacct ggaacaacag agtattgtac acatagtaca gagaccacgg aggagaagtc 360
atgaaacaaa tgcatctgga ggggacgaac cccagagcac ctcagagggc tccatatggg 420
agtccaggag cttgacacga gtggacctga gcagccatac cctgccggtg gactctgtgg 480
ggctggcggt cattctggac acagacagta agagggattc agaagcagcc agaggtccag 540
ttaaacccac ctacaacagc tttttcatct actgcaaagg cccctgccac aaggtccagc 600
ctggaaagct ccgagttcag tgtggcacct gcaaacaagc aaccctcacc ttggcccagc 660
tggctgtccc aactccctga ttaaagagct ccatcacttc aggatccttg gagaagagca 720
gtacactagg taccagcagt atggggccga ggaatgcgtg ctgcaaatgg gaggtgtgct 780
gtgcccccgt cctggctgtg gagctggact gctacctgaa cagggccaga ggaaagtcac 840
ctgcgaaggg ggcaacggcc tgggctgcgg gtttgttttc tgccgggact gtaaggaagc 900
ataccatgaa ggggattgcg actcactgct cgaaccctca ggagccactt ctcaggccta 960
cagggtggac aaaagagccg ctgagcaagc tcgctgggag gaggcctcca aggaaaccat 1020
caagaagacc accaagcctt gtcctcgctg caacgtgcca attgaaaaaa acggaggatg 1080
tatgcacatg aagtgtcctc agccccagtg caagctggag tggtgctgga actgtggctg 1140
tgagtggaac cgagcctgca tgggagatca ctggtttgac gtgtagagag agatgtcact 1200
tggccctgga cgcacaacct caagggaaac tccgaagatt cctaccttcc ttagccattt 1260
cttcttctcg atgcatataa gcacataaat gcgcacacac aaacacaggc tgcagattac 1320
agaagcagcc cctagatcct ttctagggca cccacagaaa accacagcac ccgctggccc 1380
cagggggagg aggcactttc agcctctggc tcactcgaat gtcagagctt agatgagggt 1440
gcacctttgg tttggattct gtagaagcca tgagtgaggt gggaagtgtt ttccagggtt 1500
gttgccacgc cctgggtaag taacacctct gaggattctc agaagcacac ttgagatctg 1560
aggaacgctg ctctcatgta gtaatcatct attcccaaag ggccccctgc agtagtcaaa 1620
actatttgtt tatcccccca aatcctatct ttacaaatgg tgctgatgag attacaaccc 1680
ctctgtgtac taatcagctt atcaaccaag tgagaaccta ggaaagctaa ttggatggca 1740
gactgcttaa atcgcaggga ggactcagaa gccaaaccta cttccgttcg tttcattatc 1800
tgcaacttta gaaagaaatg atcttttttt ccccctgaaa agataacaaa gtctgcaatt 1860
tggtttggag tattcctact gcagcctgga agtttagctt cactgtgaat ttaacagaga 1920
aagtgcctat aaagggggcg tttttaagag acaatcccat gatgctgcgc caatgctaac 1980
aacagggtca agaaacacaa tgtttataga aggagcatcc ctcgaccatc tgaatgagag 2040
tatgcctgac cccttccacc acaagtgggg acacctctgc atatctgctc cctcctctgc 2100
tgttaagccc cagggagccc catccaccca gtggtcctac agacagggca atacacacac 2160
accaagatag ccttcagatc aacatgcatc acactcaagt gttaatcttt caaggttttc 2220
ttttcttttt cctgtttttt atttgttttg cttttgcttt tttttttttt ttttttggtg 2280
gtggtggggc taccaaactt gaggcctaga gctaaaaatc atatagaaat gatgttatct 2340
tgtggtgtga ggaaaggcca gctggcctaa gttcacactt ttgtcccagt ggccctagac 2400
tccacccagc cagctcccaa aatgaaaaga ccacctgtca agcagcagtc aggagtctga 2460

tgtcacccat cactattttt tttccatcat tgtgcttgcc tctgcctcct tccacacccg 2520
tgtgacgtaa tcgcattggg aagccaggac aatgtttgct gttctgcttt gggtaaaggg 2580
actccctgaa gctctgtggc tctccagtat ggtccctttt ccttcctaac agatgcatat 2640
gttttcttca gaatacaata gtgattctta aaataaccca aaagacaggc atccacagtg 2700
tgtgagcatg aatcacagcc tgcattgtgt gagtgtgaat agtgggataa aagtggatgt 2760
cagaagagtg gaaatcaaac ctctgcaaag caatctttct ctttctgtga agtgtattaa 2820
gaaatacctg aagtctgtgt gtgtggtggt acccagactg tcaatcaata aagacccaga 2880
ctgtcaatga aaaaaaaaaa aaaaaaaaaa aaaaaaaa 2918

<210> 13
<211> 3043
<212> DNA
<213> mouse

<400> 13
ctcagcgagg ggaaggggga ggaggcctgg atgactaaac ctgacagaaa cgctggtggg 60
aggctcgggc gggcgccagt gcccgcgtag gtccttctcg acccgcagcc accacccgcc 120
cggtgaccat gatagtgttt gtcaggttca actccagcta tggcttccca gtggaggtcg 180
attctgacac cagcatcttg cagctcaagg aagtggttgc taagcgacag ggggttccag 240
ctgaccagct gcgtgtgatt tttgccggga aggagcttcc gaatcacctg acggttcaaa 300
actgtgacct ggaacaacag agtattgtac acatagtaca gagaccacgg aggagaagtc 360
atgaaacaaa tgcatctgga ggggacgaac cccagagcac ctcagagggc tccatatggg 420
agtccaggag cttgacacga gtggacctga gcagccatac cctgccggtg gactctgtgg 480
ggctggcggt cattctggac acagacagta agagggattc agaagcagcc agaggtccag 540
ttaaacccac ctacaacagc tttttcatct actgcaaagg cccctgccac aaggtccagc 600
ctggaaagct ccgagttcag tgtggcacct gcaaacaagc aaccctcacc ttggcccagg 660
gcccatcttg ctgggacgat gtcttaattc caaaccggat gagtggtgag tgccagtctc 720
cagactgccc tggaaccaga gctgaatttt tctttaaatg tggagcacac ccaacctcag 780
acaaggacac gtcggtagct ttgaacctga tcaccagcaa caggcgcagc atcccttgca 840
tagcgtgcac agatgtcagg agccctgtcc tggtcttcca gtgtaaccac cgtcacgtga 900
tctgtttgga ctgtttccac ttgtattgtg tcacaagact caacgatcgg cagtttgtcc 960
acgatgctca acttggctac tccctgccgt gtgtagtttg ttttctgccg ggactgtaag 1020
gaagcatacc atgaagggga ttgcgactca ctgctcgaac cctcaggagc cacttctcag 1080
gcctacaggg tggacaaaag agccgctgag caagctcgct gggaggaggc ctccaaggaa 1140
accatcaaga agaccaccaa gccttgtcct cgctgcaacg tgccaattga aaaaaacgga 1200
ggatgtatgc acatgaagtg tcctcagccc cagtgcaagc tggagtggtg ctggaactgt 1260
ggctgtgagt ggaaccgagc ctgcatggga gatcactggt ttgacgtgta gagagagatg 1320
tcacttggcc ctggacgcac aacctcaagg gaaactccga agattcctac cttccttagc 1380
catttcttct tctcgatgca tataagcaca taaatgcgca cacacaaaca caggctgcag 1440
attacagaag cagcccctag atcctttcta gggcacccac agaaaaccac agcacccgct 1500
ggccccaggg ggaggaggca ctttcagcct ctggctcact cgaatgtcag agcttagatg 1560
agggtgcacc tttggtttgg attctgtaga agccatgagt gaggtgggaa gtgttttcca 1620
gggttgttgc cacgccctgg gtaagtaaca cctctgagga ttctcagaag cacacttgag 1680
atctgaggaa cgctgctctc atgtagtaat catctattcc caaagggccc cctgcagtag 1740
tcaaaactat ttgtttatcc ccccaaatcc tatctttaca aatggtgctg atgagattac 1800
aacccctctg tgtactaatc agcttatcaa ccaagtgaga acctaggaaa gctaattgga 1860
tggcagactg cttaaatcgc agggaggact cagaagccaa acctacttcc gttcgtttca 1920
ttatctgcaa ctttagaaag aaatgatctt tttttccccc tgaaaagata acaaagtctg 1980

caatttggtt tggagtattc ctactgcagc ctggaagttt agcttcactg tgaatttaac 2040
agagaaagtg cctataaagg gggcgttttt aagagacaat cccatgatgc tgcgccaatg 2100
ctaacaacag ggtcaagaaa cacaatgttt atagaaggag catccctcga ccatctgaat 2160
gagagtatgc ctgacccctt ccaccacaag tggggacacc tctgcatatc tgctccctcc 2220
tctgctgtta agccccaggg agccccatcc acccagtggt cctacagaca gggcaataca 2280
cacacaccaa gatagccttc agatcaacat gcatcacact caagtgttaa tctttcaagg 2340
ttttcttttc tttttcctgt tttttatttg ttttgctttt gctttttttt tttttttttt 2400
tggtggtggt ggggctacca aacttgaggc ctagagctaa aaatcatata gaaatgatgt 2460
tatcttgtgg tgtgaggaaa ggccagctgg cctaagttca cacttttgtc ccagtggccc 2520
tagactccac ccagccagct cccaaaatga aaagaccacc tgtcaagcag cagtcaggag 2580
tctgatgtca cccatcacta ttttttttcc atcattgtgc ttgcctctgc ctccttccac 2640
acccgtgtga cgtaatcgca ttgggaagcc aggacaatgt ttgctgttct gctttgggta 2700
aagggactcc ctgaagctct gtggctctcc agtatggtcc cttttccttc ctaacagatg 2760
catatgtttt cttcagaata caatagtgat tcttaaaata acccaaaaga caggcatcca 2820
cagtgtgtga gcatgaatca cagcctgcat tgtgtgagtg tgaatagtgg gataaaagtg 2880
gatgtcagaa gagtggaaat caaacctctg caaagcaatc tttctctttc tgtgaagtgt 2940
attaagaaat acctgaagtc tgtgtgtgtg gtggtaccca gactgtcaat caataaagac 3000
ccagactgtc aatgaaaaaa aaaaaaaaaa aaaaaaaaaa aaa 3043

<210> 14
<211> 3253
<212> DNA
<213> mouse

<400> 14
ctcagcgagg ggaaggggga ggaggcctgg atgactaaac ctgacagaaa cgctggtggg 60
aggctcgggc gggcgccagt gcccgcgtag gtccttctcg acccgcagcc accacccgcc 120
cggtgaccat gatagtgttt gtcaggttca actccagcta tggcttccca gtggaggtcg 180
attctgacac cagcatcttg cagctcaagg aagtggttgc taagcgacgg ggttccagct 240
gaccagctgc gtgtgatttt tgccgggaag gagcttccga atcacctgac ggttcaaaac 300
tgtgacctgg aacaacagag tattgtacac atagtacaga gaccacggag gagaagtcat 360
gaaacaaatg catctggagg ggacgaaccc cagagcacct cagagggctc catatgggag 420
tccaggagct tgacacgagt ggacctgagc agccataccc tgccggtgga ctctgtgggg 480
ctggcggtca ttctggacac agacagtaag agggattcag aagcagccag aggtccagtt 540
aaacccacct acaacagctt tttcatctac tgcaaaggcc cctgccacaa ggtccagcct 600
ggaaagctcc gagttcagtg tggcacctgc aaacaagcaa ccctcacctt ggcccagggc 660
ccatcttgct gggacgatgt cttaattcca aaccggatga gtggtgagtg ccagtctcca 720
gactgccctg gaaccagagc tgaatttttc tttaaatgtg gagcacaccc aacctcagac 780
aaggacacgt cggtagcttt gaacctgatc accagcaaca ggcgcagcat cccttgcata 840
gcgtgcacag atgtcaggag ccctgtcctg gtcttccagt gtaaccaccg tcacgtgatc 900
tgtttggact gtttccactt gtattgtgtc acaagactca acgatcggca gtttgtccac 960
gatgctcaac ttggctactc cctgccgtgt gtagctggct gtcccaactc cctgattaaa 1020
gagctccatc acttcaggat ccttggagaa gagcagtaca ctaggtacca gcagtatggg 1080
gccgaggaat gcgtgctgca aatgggaggt gtgctgtgcc cccgtcctgg ctgtggagct 1140
ggactgctac ctgaacaggg ccagaggaaa gtcacctgcg aagggggcaa cggcctgggc 1200
tgcgggtttg ttttctgccg ggactgtaag gaagcatacc atgaagggga ttgcgactca 1260
ctgctcgaac cctcaggagc cacttctcag gcctacaggg tggacaaaag agccgctgag 1320
caagctcgct gggaggaggc ctccaaggaa accatcaaga agaccaccaa gccttgtcct 1380

cgctgcaacg tgccaattga aaaaaacgga ggatgtatgc acatgaagtg tcctcagccc 1440
cagtgcaagc tggagtggtg ctggaactgt ggctgtgagt ggaaccgagc ctgcatggga 1500
gatcactggt ttgacgtgta gagagagatg tcacttggcc ctggacgcac aacctcaagg 1560
gaaactccga agattcctac cttccttagc catttcttct tctcgatgca tataagcaca 1620
taaatgcgca cacacaaaca caggctgcag attacagaag cagcccctag atcctttcta 1680
gggcacccac agaaaaccac agcacccgct ggccccaggg ggaggaggca ctttcagcct 1740
ctggctcact cgaatgtcag agcttagatg agggtgcacc tttggtttgg attctgtaga 1800
agccatgagt gaggtgggaa gtgttttcca gggttgttgc cacgccctgg gtaagtaaca 1860
cctctgagga ttctcagaag cacacttgag atctgaggaa cgctgctctc atgtagtaat 1920
catctattcc caaagggccc cctgcagtag tcaaaactat ttgtttatcc ccccaaatcc 1980
tatctttaca aatggtgctg atgagattac aacccctctg tgtactaatc agcttatcaa 2040
ccaagtgaga acctaggaaa gctaattgga tggcagactg cttaaatcgc agggaggact 2100
cagaagccaa acctacttcc gttcgtttca ttatctgcaa ctttagaaag aaatgatctt 2160
tttttccccc tgaaaagata acaaagtctg caatttggtt tggagtattc ctactgcagc 2220
ctggaagttt agcttcactg tgaatttaac agagaaagtg cctataaagg gggcgttttt 2280
aagagacaat cccatgatgc tgcgccaatg ctaacaacag ggtcaagaaa cacaatgttt 2340
atagaaggag catccctcga ccatctgaat gagagtatgc ctgacccctt ccaccacaag 2400
tggggacacc tctgcatatc tgctccctcc tctgctgtta agccccaggg agccccatcc 2460
acccagtggt cctacagaca gggcaataca cacacaccaa gatagccttc agatcaacat 2520
gcatcacact caagtgttaa tctttcaagg ttttcttttc tttttcctgt tttttatttg 2580
ttttgctttt gctttttttt tttttttttt tggtggtggt ggggctacca aacttgaggc 2640
ctagagctaa aaatcatata gaaatgatgt tatcttgtgg tgtgaggaaa ggccagctgg 2700
cctaagttca cacttttgtc ccagtggccc tagactccac ccagccagct cccaaaatga 2760
aaagaccacc tgtcaagcag cagtcaggag tctgatgtca cccatcacta ttttttttcc 2820
atcattgtgc ttgcctctgc ctccttccac acccgtgtga cgtaatcgca ttgggaagcc 2880
aggacaatgt ttgctgttct gctttgggta aagggactcc ctgaagctct gtggctctcc 2940
agtatggtcc cttttccttc ctaacagatg catatgtttt cttcagaata caatagtgat 3000
tcttaaaata acccaaaaga caggcatcca cagtgtgtga gcatgaatca cagcctgcat 3060
tgtgtgagtg tgaatagtgg gataaaagtg gatgtcagaa gagtggaaat caaacctctg 3120
caaagcaatc tttctctttc tgtgaagtgt attaagaaat acctgaagtc tgtgtgtgtg 3180
gtggtaccca gactgtcaat caataaagac ccagactgtc aatgaaaaaa aaaaaaaaaa 3240
aaaaaaaaaa aaa 3253

<210> 15
<211> 3254
<212> DNA
<213> mouse

<400> 15
ctcagcgagg ggaaggggga ggaggcctgg atgactaaac ctgacagaaa cgctggtggg 60
aggctcgggc gggcgccagt gcccgcgtag gtccttctcg acccgcagcc accacccgcc 120
cggtgaccat gatagtgttt gtcaggttca actccagcta tggcttccca gtggaggtcg 180
attctgacac cagcatcttg cagctcaagg aagtggttgc taagcgacag ggggttccag 240
ctgaccagct gcgtgtgatt tttgccggga aggagcttcc gatcacctga cggttcaaaa 300
ctgtgacctg gaacaacaga gtattgtaca catagtacag agaccacgga ggagaagtca 360
tgaaacaaat gcatctggag gggacgaacc ccagagcacc tcagagggct ccatatggga 420
gtccaggagc ttgacacgag tggacctgag cagccatacc ctgccggtgg actctgtggg 480
gctggcggtc attctggaca cagacagtaa gagggattca gaagcagcca gaggtccagt 540

taaacccacc tacaacagct ttttcatcta ctgcaaaggc ccctgccaca aggtccagcc 600
tggaaagctc cgagttcagt gtggcacctg caaacaagca accctcacct tggcccaggg 660
cccatcttgc tgggacgatg tcttaattcc aaaccggatg agtggtgagt gccagtctcc 720
agactgccct ggaaccagag ctgaattttt ctttaaatgt ggagcacacc caacctcaga 780
caaggacacg tcggtagctt tgaacctgat caccagcaac aggcgcagca tcccttgcat 840
agcgtgcaca gatgtcagga gccctgtcct ggtcttccag tgtaaccacc gtcacgtgat 900
ctgtttggac tgtttccact tgtattgtgt cacaagactc aacgatcggc agtttgtcca 960
cgatgctcaa cttggctact ccctgccgtg tgtagctggc tgtcccaact ccctgattaa 1020
agagctccat cacttcagga tccttggaga agagcagtac actaggtacc agcagtatgg 1080
ggccgaggaa tgcgtgctgc aaatgggagg tgtgctgtgc ccccgtcctg gctgtggagc 1140
tggactgcta cctgaacagg gccagaggaa agtcacctgc gaagggggca acggcctggg 1200
ctgcgggttt gttttctgcc gggactgtaa ggaagcatac catgaagggg attgcgactc 1260
actgctcgaa ccctcaggag ccacttctca ggcctacagg gtggacaaaa gagccgctga 1320
gcaagctcgc tgggaggagg cctccaagga aaccatcaag aagaccacca agccttgtcc 1380
tcgctgcaac gtgccaattg aaaaaaacgg aggatgtatg cacatgaagt gtcctcagcc 1440
ccagtgcaag ctggagtggt gctggaactg tggctgtgag tggaaccgag cctgcatggg 1500
agatcactgg tttgacgtgt agagagagat gtcacttggc cctggacgca caacctcaag 1560
ggaaactccg aagattccta ccttccttag ccatttcttc ttctcgatgc atataagcac 1620
ataaatgcgc acacacaaac acaggctgca gattacagaa gcagccccta gatcctttct 1680
agggcaccca cagaaaacca cagcacccgc tggccccagg gggaggaggc actttcagcc 1740
tctggctcac tcgaatgtca gagcttagat gagggtgcac ctttggtttg gattctgtag 1800
aagccatgag tgaggtggga agtgttttcc agggttgttg ccacgccctg ggtaagtaac 1860
acctctgagg attctcagaa gcacacttga gatctgagga acgctgctct catgtagtaa 1920
tcatctattc ccaaagggcc ccctgcagta gtcaaaacta tttgtttatc cccccaaatc 1980
ctatctttac aaatggtgct gatgagatta caacccctct gtgtactaat cagcttatca 2040
accaagtgag aacctaggaa agctaattgg atggcagact gcttaaatcg cagggaggac 2100
tcagaagcca aacctacttc cgttcgtttc attatctgca actttagaaa gaaatgatct 2160
ttttttcccc ctgaaaagat aacaaagtct gcaatttggt ttggagtatt cctactgcag 2220
cctggaagtt tagcttcact gtgaatttaa cagagaaagt gcctataaag ggggcgtttt 2280
taagagacaa tcccatgatg ctgcgccaat gctaacaaca gggtcaagaa acacaatgtt 2340
tatagaagga gcatccctcg accatctgaa tgagagtatg cctgacccct tccaccacaa 2400
gtggggacac ctctgcatat ctgctccctc ctctgctgtt aagccccagg gagccccatc 2460
cacccagtgg tcctacagac agggcaatac acacacacca agatagcctt cagatcaaca 2520
tgcatcacac tcaagtgtta atctttcaag gttttctttt ctttttcctg ttttttattt 2580
gttttgcttt tgcttttttt tttttttttt ttggtggtgg tggggctacc aaacttgagg 2640
cctagagcta aaaatcatat agaaatgatg ttatcttgtg gtgtgaggaa aggccagctg 2700
gcctaagttc acacttttgt cccagtggcc ctagactcca cccagccagc tcccaaaatg 2760
aaaagaccac ctgtcaagca gcagtcagga gtctgatgtc acccatcact attttttttc 2820
catcattgtg cttgcctctg cctccttcca cacccgtgtg acgtaatcgc attgggaagc 2880
caggacaatg tttgctgttc tgctttgggt aaaggactcc cctgaagctc tgtggctctc 2940
cagtatggtc ccttttcctt cctaacagat gcatatgttt tcttcagaat acaatagtga 3000
ttcttaaaat aacccaaaag acaggcatcc acagtgtgtg agcatgaatc acagcctgca 3060
ttgtgtgagt gtgaatagtg ggataaaagt ggatgtcaga agagtggaaa tcaaacctct 3120
gcaaagcaat ctttctcttt ctgtgaagtg tattaagaaa tacctgaagt ctgtgtgtgt 3180
ggtggtaccc agactgtcaa tcaataaaga cccagactgt caatgaaaaa aaaaaaaaaa 3240
aaaaaaaaaa aaaa 3254

<210> 16

<211> 3253
<212> DNA
<213> mouse

<400> 16
ctcagcgagg ggaaggggga ggaggcctgg atgactaaac ctgacagaaa cgctggtggg 60
aggctcgggc gggcgccagt gcccgcgtag gtccttctcg acccgcagcc accacccgcc 120
cggtgaccat gatagtgttt gtcaggttca actccagcta tggcttccca gtggaggtcg 180
attctgacac cagcatcttg cagctcaagg aagtggttgc taagcgacag ggggttccag 240
ctgaccagct gcgtgtgatt tttgccggga aggagcttcc gaatcacctg acggttcaaa 300
actgtgacct ggaacaacag agtattgtac acatagtaca gagaccacgg gagaagtcat 360
gaaacaaatg catctggagg ggacgaaccc cagagcacct cagagggctc catatgggag 420
tccaggagct tgacacgagt ggacctgagc agccataccc tgccggtgga ctctgtgggg 480
ctggcggtca ttctggacac agacagtaag agggattcag aagcagccag aggtccagtt 540
aaacccacct acaacagctt tttcatctac tgcaaaggcc cctgccacaa ggtccagcct 600
ggaaagctcc gagttcagtg tggcacctgc aaacaagcaa ccctcacctt ggcccagggc 660
ccatcttgct gggacgatgt cttaattcca aaccggatga gtggtgagtg ccagtctcca 720
gactgccctg gaaccagagc tgaatttttc tttaaatgtg gagcacaccc aacctcagac 780
aaggacacgt cggtagcttt gaacctgatc accagcaaca ggcgcagcat cccttgcata 840
gcgtgcacag atgtcaggag ccctgtcctg gtcttccagt gtaaccaccg tcacgtgatc 900
tgtttggact gtttccactt gtattgtgtc acaagactca acgatcggca gtttgtccac 960
gatgctcaac ttggctactc cctgccgtgt gtagctggct gtcccaactc cctgattaaa 1020
gagctccatc acttcaggat ccttggagaa gagcagtaca ctaggtacca gcagtatggg 1080
gccgaggaat gcgtgctgca aatgggaggt gtgctgtgcc cccgtcctgg ctgtggagct 1140
ggactgctac ctgaacaggg ccagaggaaa gtcacctgcg aagggggcaa cggcctgggc 1200
tgcgggtttg ttttctgccg ggactgtaag gaagcatacc atgaagggga ttgcgactca 1260
ctgctcgaac cctcaggagc cacttctcag gcctacaggg tggacaaaag agccgctgag 1320
caagctcgct gggaggaggc ctccaaggaa accatcaaga agaccaccaa gccttgtcct 1380
cgctgcaacg tgccaattga aaaaaacgga ggatgtatgc acatgaagtg tcctcagccc 1440
cagtgcaagc tggagtggtg ctggaactgt ggctgtgagt ggaaccgagc ctgcatggga 1500
gatcactggt ttgacgtgta gagagagatg tcacttggcc ctggacgcac aacctcaagg 1560
gaaactccga agattcctac cttccttagc catttcttct tctcgatgca tataagcaca 1620
taaatgcgca cacacaaaca caggctgcag attacagaag cagcccctag atcctttcta 1680
gggcacccac agaaaaccac agcacccgct ggccccaggg ggaggaggca ctttcagcct 1740
ctggctcact cgaatgtcag agcttagatg agggtgcacc tttggtttgg attctgtaga 1800
agccatgagt gaggtgggaa gtgttttcca gggttgttgc cacgccctgg gtaagtaaca 1860
cctctgagga ttctcagaag cacacttgag atctgaggaa cgctgctctc atgtagtaat 1920
catctattcc caaagggccc cctgcagtag tcaaaactat ttgtttatcc ccccaaatcc 1980
tatctttaca aatggtgctg atgagattac aacccctctg tgtactaatc agcttatcaa 2040
ccaagtgaga acctaggaaa gctaattgga tggcagactg cttaaatcgc agggaggact 2100
cagaagccaa acctacttcc gttcgtttca ttatctgcaa ctttagaaag aaatgatctt 2160
tttttccccc tgaaaagata acaaagtctg caatttggtt tggagtattc ctactgcagc 2220
ctggaagttt agcttcactg tgaatttaac agagaaagtg cctataaagg gggcgttttt 2280
aagagacaat cccatgatgc tgcgccaatg ctaacaacag ggtcaagaaa cacaatgttt 2340
atagaaggag catccctcga ccatctgaat gagagtatgc ctgacccctt ccaccacaag 2400
tggggacacc tctgcatatc tgctccctcc tctgctgtta agccccaggg agccccatcc 2460
acccagtggt cctacagaca gggcaataca cacacaccaa gatagccttc agatcaacat 2520
gcatcacact caagtgttaa tctttcaagg ttttcttttc tttttcctgt tttttatttg 2580

ttttgctttt gctttttttt tttttttttt tggtggtggt ggggctacca aacttgaggc 2640
ctagagctaa aaatcatata gaaatgatgt tatcttgtgg tgtgaggaaa ggccagctgg 2700
cctaagttca cacttttgtc ccagtggccc tagactccac ccagccagct cccaaaatga 2760
aaagaccacc tgtcaagcag cagtcaggag tctgatgtca cccatcacta ttttttttcc 2820
atcattgtgc ttgcctctgc ctccttccac acccgtgtga cgtaatcgca ttgggaagcc 2880
aggacaatgt ttgctgttct gctttgggta aagggactcc ctgaagctct gtggctctcc 2940
agtatggtcc cttttccttc ctaacagatg catatgtttt cttcagaata caatagtgat 3000
tcttaaaata acccaaaaga caggcatcca cagtgtgtga gcatgaatca cagcctgcat 3060
tgtgtgagtg tgaatagtgg gataaaagtg gatgtcagaa gagtggaaat caaacctctg 3120
caaagcaatc tttctctttc tgtgaagtgt attaagaaat acctgaagtc tgtgtgtgtg 3180
gtggtaccca gactgtcaat caataaagac ccagactgtc aatgaaaaaa aaaaaaaaaa 3240
aaaaaaaaaa aaa 3253

<210> 17
<211> 3092
<212> DNA
<213> mouse

<400> 17
ctcagcgagg ggaaggggga ggaggcctgg atgactaaac ctgacagaaa cgctggtggg 60
aggctcgggc gggcgccagt gcccgcgtag gtccttctcg acccgcagcc accacccgcc 120
cggtgaccat gatagtaact gtgacctgga acaacagagt attgtacaca tagtacagag 180
accacggagg agaagtcatg aaacaaatgc atctggaggg gacgaacccc agagcacctc 240
agagggctcc atatgggagt ccaggagctt gacacgagtg gacctgagca gccataccct 300
gccggtggac tctgtggggc tggcggtcat tctggacaca gacagtaaga gggattcaga 360
agcagccaga ggtccagtta aacccaccta caacagcttt ttcatctact gcaaaggccc 420
ctgccacaag gtccagcctg gaaagctccg agttcagtgt ggcacctgca aacaagcaac 480
cctcaccttg gcccagggcc catcttgctg ggacgatgtc ttaattccaa accggatgag 540
tggtgagtgc cagtctccag actgccctgg aaccagagct gaatttttct ttaaatgtgg 600
agcacaccca acctcagaca aggacacgtc ggtagctttg aacctgatca ccagcaacag 660
gcgcagcatc ccttgcatag cgtgcacaga tgtcaggagc cctgtcctgg tcttccagtg 720
taaccaccgt cacgtgatct gtttggactg tttccacttg tattgtgtca caagactcaa 780
cgatcggcag tttgtccacg atgctcaact tggctactcc ctgccgtgtg tagctggctg 840
tcccaactcc ctgattaaag agctccatca cttcaggatc cttggagaag agcagtacac 900
taggtaccag cagtatgggg ccgaggaatg cgtgctgcaa atgggaggtg tgctgtgccc 960
ccgtcctggc tgtggagctg gactgctacc tgaacagggc cagaggaaag tcacctgcga 1020
agggggcaac ggcctgggct gcgggtttgt tttctgccgg gactgtaagg aagcatacca 1080
tgaaggggat tgcgactcac tgctcgaacc ctcaggagcc acttctcagg cctacagggt 1140
ggacaaaaga gccgctgagc aagctcgctg ggaggaggcc tccaaggaaa ccatcaagaa 1200
gaccaccaag ccttgtcctc gctgcaacgt gccaattgaa aaaaacggag gatgtatgca 1260
catgaagtgt cctcagcccc agtgcaagct ggagtggtgc tggaactgtg gctgtgagtg 1320
gaaccgagcc tgcatgggag atcactggtt tgacgtgtag agagagatgt cacttggccc 1380
tggacgcaca acctcaaggg aaactccgaa gattcctacc ttccttagcc atttcttctt 1440
ctcgatgcat ataagcacat aaatgcgcac acacaaacac aggctgcaga ttacagaagc 1500
agcccctaga tcctttctag ggcacccaca gaaaaccaca gcacccgctg gccccagggg 1560
gaggaggcac tttcagcctc tggctcactc gaatgtcaga gcttagatga gggtgcacct 1620
ttggtttgga ttctgtagaa gccatgagtg aggtgggaag tgttttccag ggttgttgcc 1680
acgccctggg taagtaacac ctctgaggat tctcagaagc acacttgaga tctgaggaac 1740

gctgctctca tgtagtaatc atctattccc aaagggcccc ctgcagtagt caaaactatt 1800
tgtttatccc cccaaatcct atctttacaa atggtgctga tgagattaca acccctctgt 1860
gtactaatca gcttatcaac caagtgagaa cctaggaaag ctaattggat ggcagactgc 1920
ttaaatcgca gggaggactc agaagccaaa cctacttccg ttcgtttcat tatctgcaac 1980
tttagaaaga aatgatcttt ttttccccct gaaaagataa caaagtctgc aatttggttt 2040
ggagtattcc tactgcagcc tggaagttta gcttcactgt gaatttaaca gagaaagtgc 2100
ctataaaggg ggcgttttta agagacaatc ccatgatgct gcgccaatgc taacaacagg 2160
gtcaagaaac acaatgttta tagaaggagc atccctcgac catctgaatg agagtatgcc 2220
tgaccccttc caccacaagt ggggacacct ctgcatatct gctccctcct ctgctgttaa 2280
gccccaggga gccccatcca cccagtggtc ctacagacag ggcaatacac acacaccaag 2340
atagccttca gatcaacatg catcacactc aagtgttaat ctttcaaggt tttcttttct 2400
ttttcctgtt ttttatttgt tttgcttttg cttttttttt tttttttttt ggtggtggtg 2460
gggctaccaa acttgaggcc tagagctaaa aatcatatag aaatgatgtt atcttgtggt 2520
gtgaggaaag gccagctggc ctaagttcac acttttgtcc cagtggccct agactccacc 2580
cagccagctc ccaaaatgaa aagaccacct gtcaagcagc agtcaggagt ctgatgtcac 2640
ccatcactat tttttttcca tcattgtgct tgcctctgcc tccttccaca cccgtgtgac 2700
gtaatcgcat tgggaagcca ggacaatgtt tgctgttctg ctttgggtaa agggactccc 2760
tgaagctctg tggctctcca gtatggtccc ttttccttcc taacagatgc atatgttttc 2820
ttcagaatac aatagtgatt cttaaaataa cccaaaagac aggcatccac agtgtgtgag 2880
catgaatcac agcctgcatt gtgtgagtgt gaatagtggg ataaaagtgg atgtcagaag 2940
agtggaaatc aaacctctgc aaagcaatct ttctctttct gtgaagtgta ttaagaaata 3000
cctgaagtct gtgtgtgtgg tggtacccag actgtcaatc aataaagacc cagactgtca 3060
atgaaaaaaa aaaaaaaaaa aaaaaaaaaa aa 3092

<210> 18
<211> 3255
<212> DNA
<213> mouse

<400> 18
ctcagcgagg ggaaggggga ggaggcctgg atgactaaac ctgacagaaa cgctggtggg 60
aggctcgggc gggcgccagt gcccgcgtag gtccttctcg acccgcagcc accacccgcc 120
cggtgaccat gatagtgttt gtcaggttca actccagcta tggcttccca gtggaggtcg 180
attctgacac cagcatcttg cagctcaagg aagtggttgc taagcgacag ggggttccag 240
ctgaccagct gcgtgtgatt tttgccggga aggagcttcc gaatcacctg acggttcaaa 300
actgtgacct ggaacaacag agtattgtac acatagtaca gagaccacgg aggagaagtc 360
atgaaacaaa tgcatctgga ggggacgaac cccagagcac ctcagagggc tccatatggg 420
agtccaggag cttgacacga gtggacctga gcagccatac cctgccggtg gactctgtgg 480
ggctggcggt cattctggac acagacagta agagggattc agaagcagcc agaggtccag 540
ttaaacccac ctacaacagc tttttcatct actgcaaagg cccctgccac aaggtccagc 600
ctggaaatct ccgagttcag tgtggcacct gcaaacaagc aaccctcacc ttggcccagg 660
gcccatcttg ctgggacgat gtcttaattc caaaccggat gagtggtgag tgccagtctc 720
cagactgccc tggaaccaga gctgaatttt tctttaaatg tggagcacac ccaacctcag 780
acaaggacac gtcggtagct ttgaacctga tcaccagcaa caggcgcagc atcccttgca 840
tagcgtgcac agatgtcagg agccctgtcc tggtcttcca gtgtaaccac cgtcacgtga 900
tctgtttgga ctgtttccac ttgtattgtg tcacaagact caacgatcgg cagtttgtcc 960
acgatgctca acttggctac tccctgccgt gtgtagctgg ctgtcccaac tccctgatta 1020
aagagctcca tcacttcagg atccttggag aagagcagta cactaggtac cagcagtatg 1080

gggccgagga atgcgtgctg caaatgggag gtgtgctgtg cccccgtcct ggctgtggag 1140
ctggactgct acctgaacag ggccagagga aagtcacctg cgaagggggc aacggcctgg 1200
gctgcgggtt tgttttctgc cgggactgta aggaagcata ccatgaaggg gattgcgact 1260
cactgctcga accctcagga gccacttctc aggcctacag ggtggacaaa agagccgctg 1320
agcaagctcg ctgggaggag gcctccaagg aaaccatcaa gaagaccacc aagccttgtc 1380
ctcgctgcaa cgtgccaatt gaaaaaaacg gaggatgtat gcacatgaag tgtcctcagc 1440
cccagtgcaa gctggagtgg tgctggaact gtggctgtga gtggaaccga gcctgcatgg 1500
gagatcactg gtttgacgtg tagagagaga tgtcacttgg ccctggacgc acaacctcaa 1560
gggaaactcc gaagattcct accttcctta gccatttctt cttctcgatg catataagca 1620
cataaatgcg cacacacaaa cacaggctgc agattacaga agcagcccct agatcctttc 1680
tagggcaccc acagaaaacc acagcacccg ctggccccag gggaggaggg cactttcagc 1740
ctctggctca ctcgaatgtc agagcttaga tgagggtgca cctttggttt ggattctgta 1800
gaagccatga gtgaggtggg aagtgttttc cagggttgtt gccacgccct gggtaagtaa 1860
cacctctgag gattctcaga agcacacttg agatctgagg aacgctgctc tcatgtagta 1920
atcatctatt cccaaagggc cccctgcagt agtcaaaact atttgtttat ccccccaaat 1980
cctatcttta caaatggtgc tgatgagatt acaacccctc tgtgtactaa tcagcttatc 2040
aaccaagtga gaacctagga aagctaattg gatggcagac tgcttaaatc gcagggagga 2100
ctcagaagcc aaacctactt ccgttcgttt cattatctgc aactttagaa agaaatgatc 2160
tttttttccc cctgaaaaga taacaaagtc tgcaatttgg tttggagtat tcctactgca 2220
gcctggaagt ttagcttcac tgtgaattta acagagaaag tgcctataaa gggggcgttt 2280
ttaagagaca atcccatgat gctgcgccaa tgctaacaac agggtcaaga aacacaatgt 2340
ttatagaagg agcatccctc gaccatctga atgagagtat gcctgacccc ttccaccaca 2400
agtggggaca cctctgcata tctgctccct cctctgctgt taagccccag ggagccccat 2460
ccacccagtg gtcctacaga cagggcaata cacacacacc aagatagcct tcagatcaac 2520
atgcatcaca ctcaagtgtt aatctttcaa ggttttcttt tctttttcct gttttttatt 2580
tgttttgctt ttgctttttt tttttttttt tttggtggtg gtggggctac caaacttgag 2640
gcctagagct aaaaatcata tagaaatgat gttatcttgt ggtgtgagga aaggccagct 2700
ggcctaagtt cacacttttg tcccagtggc cctagactcc acccagccag ctcccaaaat 2760
gaaaagacca cctgtcaagc agcagtcagg agtctgatgt cacccatcac tatttttttt 2820
ccatcattgt gcttgcctct gcctccttcc acacccgtgt gacgtaatcg cattgggaag 2880
ccaggacaat gtttgctgtt ctgctttggg taaaggactc ccctgaagct ctgtggctct 2940
ccagtatggt cccttttcct tcctaacaga tgcatatgtt ttcttcagaa tacaatagtg 3000
attcttaaaa taacccaaaa gacaggcatc cacagtgtgt gagcatgaat cacagcctgc 3060
attgtgtgag tgtgaatagt gggataaaag tggatgtcag aagagtggaa atcaaacctc 3120
tgcaaagcaa tctttctctt tctgtgaagt gtattaagaa atacctgaag tctgtgtgtg 3180
tggtggtacc cagactgtca atcaataaag acccagactg tcaatgaaaa aaaaaaaaaa 3240
aaaaaaaaaa aaaaa 3255

<210> 19
<211> 3255
<212> DNA
<213> mouse

<400> 19
ctcagcgagg ggaaggggga ggaggcctgg atgactaaac ctgacagaaa cgctggtggg 60
aggctcgggc gggcgccagt gcccgcgtag gtccttctcg acccgcagcc accacccgcc 120
cggtgaccat gatagtgttt gtcaggttca actccagcta tggcttccca gtggaggtcg 180
attctgacac cagcatcttg cagctcaagg aagtggttgc taagcgacag ggggttccag 240

ctgaccagct gcgtgtgatt tttgccggga aggagcttcc gaatcacctg acggttcaaa 300
actgtgacct ggaacaacag agtattgtac acatagtaca gagaccacgg aggagaagtc 360
atgaaacaaa tgcatctgga ggggacgaac cccagagcac ctcagagggc tccatatggg 420
agtccaggag cttgacacga gtggacctga gcagccatac cctgccggtg gactctgtgg 480
ggctggcggt cattctggac acagacagta agagggattc agaagcagcc agaggtccag 540
ttaaacccac ctacaacagc tttttcatct actgcaaagg cccctgccac aaggtccagc 600
ctggaaagct ccgagttcag tgtggcacct gcaaacaagc aaccctcacc ttggcccagg 660
gcccatcttg ctgggacgat gtcttaattc caaaccggat gagtggtgag tgccagtctc 720
cagactgccc tggaaccaga gctgaatttt tctttaaatg tggagcacac ccaacctcag 780
acaaggacac gtcggtagct ttgaacctga tcaccagcaa caggcgcagc atcccttgca 840
tagcgtgcac agatgtcagg agccctgtcc tggtcttcca gtgtaaccac cgtcacgtga 900
tctgtttgga ctgtttccac ttgtattgtg tcacaagact caacgatcgg cagtttgtcc 960
acgatgctca acttggctac tccctgccgt gtgtagctgg ctgtcccaac tccctgatta 1020
aagagctcca tcacttcagg atccttggag aagagcagta cactaggtac cagcagtatg 1080
gggccgagga atgcgtgctg caaatgggag gtgtgctgtg cccccgtcct ggctgtggag 1140
ctggactgct acctgaacag ggccagagga aagtcacctg cgaagggggc aacggcctgg 1200
gctgcgggtt tgttttctgc cgggactgta aggaagcata ccatgaaggg gattgcgact 1260
cactgctcga accctcagga gccacttctc aggcctacag ggtggacaaa agagccgctg 1320
agcaagctcg ctgggaggag gcctccaagg aaaccatcaa gaagaccaac aagccttgtc 1380
ctcgctgcaa cgtgccaatt gaaaaaaacg gaggatgtat gcacatgaag tgtcctcagc 1440
cccagtgcaa gctggagtgg tgctggaact gtggctgtga gtggaaccga gcctgcatgg 1500
gagatcactg gtttgacgtg tagagagaga tgtcacttgg ccctggacgc acaacctcaa 1560
gggaaactcc gaagattcct accttcctta gccatttctt cttctcgatg catataagca 1620
cataaatgcg cacacacaaa cacaggctgc agattacaga agcagcccct agatcctttc 1680
tagggcaccc acagaaaacc acagcacccg ctggccccag ggggaggagg cactttcagc 1740
ctctggctca ctcgaatgtc agagcttaga tgagggtgca cctttggttt ggattctgta 1800
gaagccatga gtgaggtggg aagtgttttc caggttgttt gccacgccct gggtaagtaa 1860
cacctctgag gattctcaga agcacacttg agatctgagg aacgctgctc tcatgtagta 1920
atcatctatt cccaaagggc cccctgcagt agtcaaaact atttgtttat ccccccaaat 1980
cctatcttta caaatggtgc tgatgagatt acaacccctc tgtgtactaa tcagcttatc 2040
aaccaagtga gaacctagga aagctaattg gatggcagac tgcttaaatc gcagggagga 2100
ctcagaagcc aaacctactt ccgttcgttt cattatctgc aactttagaa agaaatgatc 2160
tttttttccc cctgaaaaga taacaaagtc tgcaatttgg tttggagtat tcctactgca 2220
gcctggaagt ttagcttcac tgtgaattta acagagaaag tgcctataaa gggggcgttt 2280
ttaagagaca atcccatgat gctgcgccaa tgctaacaac agggtcaaga aacacaatgt 2340
ttatagaagg agcatccctc gaccatctga atgagagtat gcctgacccc ttccaccaca 2400
agtggggaca cctctgcata tctgctccct cctctgctgt taagccccag ggagccccat 2460
ccacccagtg gtcctacaga cagggcaata cacacacacc aagatagcct tcagatcaac 2520
atgcatcaca ctcaagtgtt aatctttcaa ggttttcttt tctttttcct gttttttatt 2580
tgttttgctt ttgctttttt tttttttttt tttggtggtg gtggggctac caaacttgag 2640
gcctagagct aaaaatcata tagaaatgat gttatcttgt ggtgtgagga aaggccagct 2700
ggcctaagtt cacacttttg tcccagtggc cctagactcc acccagccag ctcccaaaat 2760
gaaaagacca cctgtcaagc agcagtcagg agtctgatgt cacccatcac tatttttttt 2820
ccatcattgt gcttgcctct gcctccttcc acacccgtgt gacgtaatcg cattgggaag 2880
ccaggacaat gtttgctgtt ctgctttggg taaagggact ccctgaagct ctgtggctct 2940
ccagtatggt cccttttcct tcctaacaga tgcatatgtt ttcttcagaa tacaatagtg 3000
attcttaaaa taacccaaaa gacaggcatc cacagtgtgt gagcatgaat cacagcctgc 3060
attgtgtgag tgtgaatagt gggataaaag tggatgtcag aagagtggaa atcaaacctc 3120

tgcaaagcaa tctttctctt tctgtgaagt gtattaagaa atacctgaag tctgtgtgtg 3180
tggtggtacc cagactgtca atcaataaag acccagactg tcaatgaaaa aaaaaaaaaa 3240
aaaaaaaaaa aaaaa 3255

<210> 20
<211> 3255
<212> DNA
<213> mouse

<400> 20
ctcagcgagg ggaaggggga ggaggcctgg atgactaaac ctgacagaaa cgctggtggg 60
aggctcgggc gggcgccagt gcccgcgtag gtccttctcg acccgcagcc accacccgcc 120
cggtgaccat gatagtgttt gtcaggttca actccagcta tggcttccca gtggaggtcg 180
attctgacac cagcatcttg cagctcaagg aagtggttgc taagcgacag ggggttccag 240
ctgaccagct gcgtgtgatt tttgccggga aggagcttcc gaatcacctg acggttcaaa 300
actgtgacct ggaacaacag agtattgtac acatagtaca gagaccacgg aggagaagtc 360
atgaaacaaa tgcatctgga ggggacgaac cccagagcac ctcagagggc tccatatggg 420
agtccaggag cttgacacga gtggacctga gcagccatac cctgccggtg gactctgtgg 480
ggctggcggt cattctggac acagacagta agagggattc agaagcagcc agaggtccag 540
ttaaacccac ctacaacagc tttttcatct actgcaaagg cccctgccac aaggtccagc 600
ctggaaagct ccgagttcag tgtggcacct gcaaacaagc aaccctcacc ttggcccagg 660
gcccatcttg ctgggacgat gtcttaattc caaaccggat gagtggtgag tgccagtctc 720
cagactgccc tggaaccaga gctgaatttt tctttaaatg tggagcacac ccaacctcag 780
acaaggacac gtcggtagct ttgaacctga tcaccagcaa caggcgcagc atcccttgca 840
tagcgtgcac agatgtcagg agccctgtcc tggtcttcca gtgtaaccac cgtcacgtga 900
tctgtttgga ctgtttccac ttgtattgtg tcacaagact caacgatcgg cagtttgtcc 960
acgatgctca acttggctac tccctgccgt gtgtagctgg ctgtcccaac tccctgatta 1020
aagagctcca tcacttcagg atccttggag aagagcagta cactaggtac cagcagtatg 1080
gggccgagga atgcgtgctg caaatgggag gtgtgctgtg cccccgtcct ggctgtggag 1140
ctggactgct acctgaacag ggccagagga aagtcacctg cgaagggggc aacggcctgg 1200
gctgcgggtt tgttttctgc cgggactgta aggaagcata ccatgaaggg gattgcgact 1260
cactgctcga accctcagga gccacttctc aggcctacag ggtggacaaa agagccgctg 1320
agcaagctcg ctgggaggag gcctccaagg aaaccatcaa gaagaccacc aagccttgtc 1380
ctcgctgcaa cgtgccaatt gaaaaaaacg gaggatgtat gcacatgaag tgtcctcagc 1440
cccagtgcaa gctggagtgg tgctggaact gtggctgtga gtagaaccga gcctgcatgg 1500
gagatcactg gtttgacgtg tagagagaga tgtcacttgg ccctggacgc acaacctcaa 1560
gggaaactcc gaagattcct accttcctta gccatttctt cttctcgatg catataagca 1620
cataaatgcg cacacacaaa cacaggctgc agattacaga agcagcccct agatcctttc 1680
tagggcaccc acagaaaacc acagcacccg ctggccccag gggaggaggg cactttcagc 1740
ctctggctca ctcgaatgtc agagcttaga tgaggtgcaa cctttggttt ggattctgta 1800
gaagccatga gtgaggtggg aagtgttttc cagggttgtt gccacgccct gggtaagtaa 1860
cacctctgag gattctcaga agcacacttg agatctgagg aacgctgctc tcatgtagta 1920
atcatctatt cccaaagggc cccctgcagt agtcaaaact atttgtttat ccccccaaat 1980
cctatcttta caaatggtgc tgatgagatt acaacccctc tgtgtactaa tcagcttatc 2040
aaccaagtga gaacctagga aagctaattg gatggcagac tgcttaaatc gcagggagga 2100
ctcagaagcc aaacctactt ccgttcgttt cattatctgc aactttagaa agaaatgatc 2160
tttttttccc cctgaaaaga taacaaagtc tgcaatttgg tttggagtat tcctactgca 2220
gcctggaagt ttagcttcac tgtgaattta acagagaaag tgcctataaa gggggcgttt 2280

ttaagagaca atcccatgat gctgcgccaa tgctaacaac agggtcaaga aacacaatgt 2340
ttatagaagg agcatccctc gaccatctga atgagagtat gcctgacccc ttccaccaca 2400
agtggggaca cctctgcata tctgctccct cctctgctgt taagccccag ggagccccat 2460
ccacccagtg gtcctacaga cagggcaata cacacacacc aagatagcct tcagatcaac 2520
atgcatcaca ctcaagtgtt aatctttcaa ggttttcttt tctttttcct gttttttatt 2580
tgttttgctt ttgctttttt tttttttttt tttggtggtg gtggggctac caaacttgag 2640
gcctagagct aaaaatcata tagaaatgat gttatcttgt ggtgtgagga aaggccagct 2700
ggcctaagtt cacacttttg tcccagtggc cctagactcc acccagccag ctcccaaaat 2760
gaaaagacca cctgtcaagc agcagtcagg agtctgatgt cacccatcac tatttttttt 2820
ccatcattgt gcttgcctct gcctccttcc acacccgtgt gacgtaatcg cattgggaag 2880
ccaggacaat gtttgctgtt ctgctttggg taaagggact ccctgaagct ctgtggctct 2940
ccagtatggt cccttttcct tcctaacaga tgcatatgtt ttcttcagaa tacaatagtg 3000
attcttaaaa taacccaaaa gacaggcatc cacagtgtgt gagcatgaat cacagcctgc 3060
attgtgtgag tgtgaatagt gggataaaag tggatgtcag aagagtggaa atcaaacctc 3120
tgcaaagcaa tctttctctt tctgtgaagt gtattaagaa atacctgaag tctgtgtgtg 3180
tggtggtacc cagactgtca atcaataaag acccagactg tcaatgaaaa aaaaaaaaaa 3240
aaaaaaaaaa aaaaa 3255

<210> 21
<211> 105
<212> PRT
<213> mouse

<400> 21
Met Ile Val Phe Val Arg Phe Asn Ser Ser Tyr Gly Phe Pro Val Glu
1               5                   10                  15

Val Asp Ser Asp Thr Ser Ile Leu Gln Leu Lys Glu Val Val Ala Lys
            20                  25                  30

Arg Gln Gly Val Pro Ala Asp Gln Leu Arg Val Ile Phe Ala Gly Lys
        35                  40                  45

Glu Leu Pro Asn His Leu Thr Val Gln Leu Asn Pro Pro Thr Thr Ala
    50                  55                  60

Phe Ser Ser Thr Ala Lys Ala Pro Ala Thr Arg Ser Ser Leu Glu Ser
65                  70                  75                  80

Ser Glu Phe Ser Val Ala Pro Ala Asn Lys Gln Pro Ser Pro Trp Pro
                85                  90                  95

Arg Ala His Leu Ala Gly Thr Met Ser
            100                 105

<210> 22

<211> 344
<212> PRT
<213> mouse

<400> 22
Met Ile Val Phe Val Arg Phe Asn Ser Ser Tyr Gly Phe Pro Val Glu
1               5                   10                  15

Val Asp Ser Asp Thr Ser Ile Leu Gln Leu Lys Glu Val Val Ala Lys
            20                  25                  30

Arg Gln Gly Val Pro Ala Asp Gln Leu Arg Val Ile Phe Ala Gly Lys
        35                  40                  45

Glu Leu Pro Asn His Leu Thr Val Gln Gly Pro Ser Cys Trp Asp Asp
    50                  55                  60

Val Leu Ile Pro Asn Arg Met Ser Gly Glu Cys Gln Ser Pro Asp Cys
65                  70                  75                  80

Pro Gly Thr Arg Ala Glu Phe Phe Phe Lys Cys Gly Ala His Pro Thr
                85                  90                  95

Ser Asp Lys Asp Thr Ser Val Ala Leu Asn Leu Ile Thr Ser Asn Arg
            100                 105                 110

Arg Ser Ile Pro Cys Ile Ala Cys Thr Asp Val Arg Ser Pro Val Leu
        115                 120                 125

Val Phe Gln Cys Asn His Arg His Val Ile Cys Leu Asp Cys Phe His
    130                 135                 140

Leu Tyr Cys Val Thr Arg Leu Asn Asp Arg Gln Phe Val His Asp Ala
145                 150                 155                 160

Gln Leu Gly Tyr Ser Leu Pro Cys Val Ala Gly Cys Pro Asn Ser Leu
                165                 170                 175

Ile Lys Glu Leu His His Phe Arg Ile Leu Gly Glu Glu Gln Tyr Thr
            180                 185                 190

Arg Tyr Gln Gln Tyr Gly Ala Glu Glu Cys Val Leu Gln Met Gly Gly
        195                 200                 205

Val Leu Cys Pro Arg Pro Gly Cys Gly Ala Gly Leu Leu Pro Glu Gln
    210                 215                 220

Gly Gln Arg Lys Val Thr Cys Glu Gly Gly Asn Gly Leu Gly Cys Gly

225                 230                 235                 240

Phe Val Phe Cys Arg Asp Cys Lys Glu Ala Tyr His Glu Gly Asp Cys
                245                 250                 255

Asp Ser Leu Leu Glu Pro Ser Gly Ala Thr Ser Gln Ala Tyr Arg Val
            260                 265                 270

Asp Lys Arg Ala Ala Glu Gln Ala Arg Trp Glu Glu Ala Ser Lys Glu
        275                 280                 285

Thr Ile Lys Lys Thr Thr Lys Pro Cys Pro Arg Cys Asn Val Pro Ile
    290                 295                 300

Glu Lys Asn Gly Gly Cys Met His Met Lys Cys Pro Gln Pro Gln Cys
305                 310                 315                 320

Lys Leu Glu Trp Cys Trp Asn Cys Gly Cys Glu Trp Asn Arg Ala Cys
                325                 330                 335

Met Gly Asp His Trp Phe Asp Val
            340

<210> 23
<211> 63
<212> PRT
<213> mouse

<400> 23
Met Ile Val Phe Val Arg Phe Asn Ser Ser Tyr Gly Phe Pro Val Glu
  1               5                  10                  15

Val Asp Ser Asp Thr Ser Ile Leu Gln Leu Lys Glu Val Val Ala Lys
             20                  25                  30

Arg Gln Gly Val Pro Ala Asp Gln Leu Arg Val Ile Phe Ala Gly Lys
         35                  40                  45

Glu Leu Pro Asn His Leu Thr Val Gln Leu Ala Val Pro Thr Pro
     50                  55                  60

<210> 24
<211> 153
<212> PRT
<213> mouse

<400> 24
Met Ile Val Phe Val Arg Phe Asn Ser Ser Tyr Gly Phe Pro Val Glu
1               5                   10                  15

Val Asp Ser Asp Thr Ser Ile Leu Gln Leu Lys Glu Val Val Ala Lys
            20                  25                  30

Arg Gln Gly Val Pro Ala Asp Gln Leu Arg Val Ile Phe Ala Gly Lys
        35                  40                  45

Glu Leu Pro Asn His Leu Thr Val Gln Asn Cys Asp Leu Glu Gln Gln
    50                  55                  60

Ser Ile Val His Ile Val Gln Arg Pro Arg Arg Arg Ser His Glu Thr
65                  70                  75                  80

Asn Ala Ser Gly Gly Asp Glu Pro Gln Ser Thr Ser Glu Gly Ser Ile
                85                  90                  95

Trp Glu Ser Arg Ser Leu Thr Arg Val Asp Leu Ser Ser His Thr Leu
            100                 105                 110

Pro Val Asp Ser Val Gly Leu Ala Val Ile Leu Asp Thr Asp Ser Lys
        115                 120                 125

Arg Asp Ser Glu Ala Ala Arg Gly Pro Gly Pro Ile Leu Leu Gly Arg
    130                 135                 140

Cys Leu Asn Ser Lys Pro Asp Glu Trp
145                 150


<210> 25
<211> 194
<212> PRT
<213> mouse

<400> 25
Met Ile Val Phe Val Arg Phe Asn Ser Ser Tyr Gly Phe Pro Val Glu
1               5                   10                  15

Val Asp Ser Asp Thr Ser Ile Leu Gln Leu Lys Glu Val Val Ala Lys
            20                  25                  30

Arg Gln Gly Val Pro Ala Asp Gln Leu Arg Val Ile Phe Ala Gly Lys
        35                  40                  45

Glu Leu Pro Asn His Leu Thr Val Gln Asn Cys Asp Leu Glu Gln Gln

50                  55                  60

Ser Ile Val His Ile Val Gln Arg Pro Arg Arg Arg Ser His Glu Thr
65                  70                  75                  80

Asn Ala Ser Gly Gly Asp Glu Pro Gln Ser Thr Ser Glu Gly Ser Ile
                85                  90                  95

Trp Glu Ser Arg Ser Leu Thr Arg Val Asp Leu Ser Ser His Thr Leu
            100                 105                 110

Pro Val Asp Ser Val Gly Leu Ala Val Ile Leu Asp Thr Asp Ser Lys
        115                 120                 125

Arg Asp Ser Glu Ala Ala Arg Gly Pro Val Lys Pro Thr Tyr Asn Ser
    130                 135                 140

Phe Phe Ile Tyr Cys Lys Gly Pro Cys His Lys Val Gln Pro Gly Lys
145                 150                 155                 160

Leu Arg Val Gln Cys Gly Thr Cys Lys Gln Ala Thr Leu Thr Leu Ala
                165                 170                 175

Gln Asn Phe Ser Leu Asn Val Glu His Thr Gln Pro Gln Thr Arg Thr
            180                 185                 190

Arg Arg

<210> 26
<211> 183
<212> PRT
<213> mouse

<400> 26
Met Ile Val Phe Val Arg Phe Asn Ser Ser Tyr Gly Phe Pro Val Glu
1               5                   10                  15

Val Asp Ser Asp Thr Ser Ile Leu Gln Leu Lys Glu Val Val Ala Lys
            20                  25                  30

Arg Gln Gly Val Pro Ala Asp Gln Leu Arg Val Ile Phe Ala Gly Lys
        35                  40                  45

Glu Leu Pro Asn His Leu Thr Val Gln Asn Cys Asp Leu Glu Gln Gln
    50                  55                  60

Ser Ile Val His Ile Val Gln Arg Pro Arg Arg Arg Ser His Glu Thr
65                  70                  75                  80

Asn Ala Ser Gly Gly Asp Glu Pro Gln Ser Thr Ser Glu Gly Ser Ile
                85                  90                  95

Trp Glu Ser Arg Ser Leu Thr Arg Val Asp Leu Ser Ser His Thr Leu
            100                 105                 110

Pro Val Asp Ser Val Gly Leu Ala Val Ile Leu Asp Thr Asp Ser Lys
        115                 120                 125

Arg Asp Ser Glu Ala Ala Arg Gly Pro Val Lys Pro Thr Tyr Asn Ser
    130                 135                 140

Phe Phe Ile Tyr Cys Lys Gly Pro Cys His Lys Val Gln Pro Gly Lys
145                 150                 155                 160

Leu Arg Val Gln Cys Gly Thr Cys Lys Gln Ala Thr Leu Thr Leu Ala
                165                 170                 175

Gln Leu Ala Val Pro Thr Pro
            180

<210> 27
<211> 296
<212> PRT
<213> mouse

<400> 27
Met Ile Val Phe Val Arg Phe Asn Ser Ser Tyr Gly Phe Pro Val Glu
  1               5                  10                  15

Val Asp Ser Asp Thr Ser Ile Leu Gln Leu Lys Glu Val Val Ala Lys
             20                  25                  30

Arg Gln Gly Val Pro Ala Asp Gln Leu Arg Val Ile Phe Ala Gly Lys
         35                  40                  45

Glu Leu Pro Asn His Leu Thr Val Gln Asn Cys Asp Leu Glu Gln Gln
     50                  55                  60

Ser Ile Val His Ile Val Gln Arg Pro Arg Arg Arg Ser His Glu Thr
 65                  70                  75                  80

Asn Ala Ser Gly Gly Asp Glu Pro Gln Ser Thr Ser Glu Gly Ser Ile
                 85                  90                  95

Trp Glu Ser Arg Ser Leu Thr Arg Val Asp Leu Ser Ser His Thr Leu
            100                 105                 110

Pro Val Asp Ser Val Gly Leu Ala Val Ile Leu Asp Thr Asp Ser Lys
        115                 120                 125

Arg Asp Ser Glu Ala Ala Arg Gly Pro Val Lys Pro Thr Tyr Asn Ser
    130                 135                 140

Phe Phe Ile Tyr Cys Lys Gly Pro Cys His Lys Val Gln Pro Gly Lys
145                 150                 155                 160

Leu Arg Val Gln Cys Gly Thr Cys Lys Gln Ala Thr Leu Thr Leu Ala
                165                 170                 175

Gln Gly Pro Ser Cys Trp Asp Asp Val Leu Ile Pro Asn Arg Met Ser
            180                 185                 190

Gly Glu Cys Gln Ser Pro Asp Cys Pro Gly Thr Arg Ala Glu Phe Phe
        195                 200                 205

Phe Lys Cys Gly Ala His Pro Thr Ser Asp Lys Asp Thr Ser Val Ala
    210                 215                 220

Leu Asn Leu Ile Thr Ser Asn Arg Arg Ser Ile Pro Cys Ile Ala Cys
225                 230                 235                 240

Thr Asp Val Arg Ser Pro Val Leu Val Phe Gln Cys Asn His Arg His
                245                 250                 255

Val Ile Cys Leu Asp Cys Phe His Leu Tyr Cys Val Thr Arg Leu Asn
            260                 265                 270

Asp Arg Gln Phe Val His Asp Ala Gln Leu Gly Tyr Ser Leu Pro Cys
        275                 280                 285

Val Val Cys Phe Leu Pro Gly Leu
    290                 295

<210> 28
<211> 37
<212> PRT
<213> mouse

<400> 28
Met Ile Val Phe Val Arg Phe Asn Ser Ser Tyr Gly Phe Pro Val Glu

1               5                   10                  15

Val Asp Ser Asp Thr Ser Ile Leu Gln Leu Lys Glu Val Val Ala Lys
            20                  25                  30

Arg Arg Gly Ser Ser
        35

<210> 29
<211> 53
<212> PRT
<213> mouse

<400> 29
Met Ile Val Phe Val Arg Phe Asn Ser Ser Tyr Gly Phe Pro Val Glu
1               5                   10                  15

Val Asp Ser Asp Thr Ser Ile Leu Gln Leu Lys Glu Val Val Ala Lys
            20                  25                  30

Arg Gln Gly Val Pro Ala Asp Gln Leu Arg Val Ile Phe Ala Gly Lys
        35                  40                  45

Glu Leu Pro Ile Thr
    50

<210> 30
<211> 77
<212> PRT
<213> mouse

<400> 30
Met Ile Val Phe Val Arg Phe Asn Ser Ser Tyr Gly Phe Pro Val Glu
1               5                   10                  15

Val Asp Ser Asp Thr Ser Ile Leu Gln Leu Lys Glu Val Val Ala Lys
            20                  25                  30

Arg Gln Gly Val Pro Ala Asp Gln Leu Arg Val Ile Phe Ala Gly Lys
        35                  40                  45

Glu Leu Pro Asn His Leu Thr Val Gln Asn Cys Asp Leu Glu Gln Gln
    50                  55                  60

Ser Ile Val His Ile Val Gln Arg Pro Arg Glu Lys Ser
65                  70                  75

<210> 31
<211> 14
<212> PRT
<213> mouse

<400> 31
Met Ile Val Thr Val Thr Trp Asn Asn Arg Val Leu Tyr Thr
1               5                   10

<210> 32
<211> 464
<212> PRT
<213> mouse

<400> 32
Met Ile Val Phe Val Arg Phe Asn Ser Ser Tyr Gly Phe Pro Val Glu
1               5                   10                  15

Val Asp Ser Asp Thr Ser Ile Leu Gln Leu Lys Glu Val Val Ala Lys
            20                  25                  30

Arg Gln Gly Val Pro Ala Asp Gln Leu Arg Val Ile Phe Ala Gly Lys
        35                  40                  45

Glu Leu Pro Asn His Leu Thr Val Gln Asn Cys Asp Leu Glu Gln Gln
    50                  55                  60

Ser Ile Val His Ile Val Gln Arg Pro Arg Arg Arg Ser His Glu Thr
65                  70                  75                  80

Asn Ala Ser Gly Gly Asp Glu Pro Gln Ser Thr Ser Glu Gly Ser Ile
                85                  90                  95

Trp Glu Ser Arg Ser Leu Thr Arg Val Asp Leu Ser Ser His Thr Leu
            100                 105                 110

Pro Val Asp Ser Val Gly Leu Ala Val Ile Leu Asp Thr Asp Ser Lys
        115                 120                 125

Arg Asp Ser Glu Ala Ala Arg Gly Pro Val Lys Pro Thr Tyr Asn Ser
    130                 135                 140

Phe Phe Ile Tyr Cys Lys Gly Pro Cys His Lys Val Gln Pro Gly Asn
145                 150                 155                 160

Leu Arg Val Gln Cys Gly Thr Cys Lys Gln Ala Thr Leu Thr Leu Ala
                165                 170                 175

Gln Gly Pro Ser Cys Trp Asp Asp Val Leu Ile Pro Asn Arg Met Ser
            180                 185                 190

Gly Glu Cys Gln Ser Pro Asp Cys Pro Gly Thr Arg Ala Glu Phe Phe
        195                 200                 205

Phe Lys Cys Gly Ala His Pro Thr Ser Asp Lys Asp Thr Ser Val Ala
    210                 215                 220

Leu Asn Leu Ile Thr Ser Asn Arg Arg Ser Ile Pro Cys Ile Ala Cys
225                 230                 235                 240

Thr Asp Val Arg Ser Pro Val Leu Val Phe Gln Cys Asn His Arg His
                245                 250                 255

Val Ile Cys Leu Asp Cys Phe His Leu Tyr Cys Val Thr Arg Leu Asn
            260                 265                 270

Asp Arg Gln Phe Val His Asp Ala Gln Leu Gly Tyr Ser Leu Pro Cys
        275                 280                 285

Val Ala Gly Cys Pro Asn Ser Leu Ile Lys Glu Leu His His Phe Arg
    290                 295                 300

Ile Leu Gly Glu Glu Gln Tyr Thr Arg Tyr Gln Gln Tyr Gly Ala Glu
305                 310                 315                 320

Glu Cys Val Leu Gln Met Gly Gly Val Leu Cys Pro Arg Pro Gly Cys
                325                 330                 335

Gly Ala Gly Leu Leu Pro Glu Gln Gly Gln Arg Lys Val Thr Cys Glu
            340                 345                 350

Gly Gly Asn Gly Leu Gly Cys Gly Phe Val Phe Cys Arg Asp Cys Lys
        355                 360                 365

Glu Ala Tyr His Glu Gly Asp Cys Asp Ser Leu Leu Glu Pro Ser Gly
    370                 375                 380

Ala Thr Ser Gln Ala Tyr Arg Val Asp Lys Arg Ala Ala Glu Gln Ala
385                 390                 395                 400

Arg Trp Glu Glu Ala Ser Lys Glu Thr Ile Lys Lys Thr Thr Lys Pro
                405                 410                 415

Cys Pro Arg Cys Asn Val Pro Ile Glu Lys Asn Gly Gly Cys Met His
            420                 425                 430

Met Lys Cys Pro Gln Pro Gln Cys Lys Leu Glu Trp Cys Trp Asn Cys
        435                 440                 445

Gly Cys Glu Trp Asn Arg Ala Cys Met Gly Asp His Trp Phe Asp Val
    450                 455                 460

<210> 33
<211> 464
<212> PRT
<213> mouse

<400> 33
Met Ile Val Phe Val Arg Phe Asn Ser Ser Tyr Gly Phe Pro Val Glu
1               5                   10                  15

Val Asp Ser Asp Thr Ser Ile Leu Gln Leu Lys Glu Val Val Ala Lys
            20                  25                  30

Arg Gln Gly Val Pro Ala Asp Gln Leu Arg Val Ile Phe Ala Gly Lys
        35                  40                  45

Glu Leu Pro Asn His Leu Thr Val Gln Asn Cys Asp Leu Glu Gln Gln
    50                  55                  60

Ser Ile Val His Ile Val Gln Arg Pro Arg Arg Arg Ser His Glu Thr
65                  70                  75                  80

Asn Ala Ser Gly Gly Asp Glu Pro Gln Ser Thr Ser Glu Gly Ser Ile
                85                  90                  95

Trp Glu Ser Arg Ser Leu Thr Arg Val Asp Leu Ser Ser His Thr Leu
            100                 105                 110

Pro Val Asp Ser Val Gly Leu Ala Val Ile Leu Asp Thr Asp Ser Lys
        115                 120                 125

Arg Asp Ser Glu Ala Ala Arg Gly Pro Val Lys Pro Thr Tyr Asn Ser
    130                 135                 140

Phe Phe Ile Tyr Cys Lys Gly Pro Cys His Lys Val Gln Pro Gly Lys
145                 150                 155                 160

Leu Arg Val Gln Cys Gly Thr Cys Lys Gln Ala Thr Leu Thr Leu Ala
                165                 170                 175

Gln Gly Pro Ser Cys Trp Asp Asp Val Leu Ile Pro Asn Arg Met Ser
            180                 185                 190

Gly Glu Cys Gln Ser Pro Asp Cys Pro Gly Thr Arg Ala Glu Phe Phe
        195                 200                 205

Phe Lys Cys Gly Ala His Pro Thr Ser Asp Lys Asp Thr Ser Val Ala
    210                 215                 220

Leu Asn Leu Ile Thr Ser Asn Arg Arg Ser Ile Pro Cys Ile Ala Cys
225                 230                 235                 240

Thr Asp Val Arg Ser Pro Val Leu Val Phe Gln Cys Asn His Arg His
                245                 250                 255

Val Ile Cys Leu Asp Cys Phe His Leu Tyr Cys Val Thr Arg Leu Asn
            260                 265                 270

Asp Arg Gln Phe Val His Asp Ala Gln Leu Gly Tyr Ser Leu Pro Cys
        275                 280                 285

Val Ala Gly Cys Pro Asn Ser Leu Ile Lys Glu Leu His His Phe Arg
    290                 295                 300

Ile Leu Gly Glu Glu Gln Tyr Thr Arg Tyr Gln Gln Tyr Gly Ala Glu
305                 310                 315                 320

Glu Cys Val Leu Gln Met Gly Gly Val Leu Cys Pro Arg Pro Gly Cys
                325                 330                 335

Gly Ala Gly Leu Leu Pro Glu Gln Gly Gln Arg Lys Val Thr Cys Glu
            340                 345                 350

Gly Gly Asn Gly Leu Gly Cys Gly Phe Val Phe Cys Arg Asp Cys Lys
        355                 360                 365

Glu Ala Tyr His Glu Gly Asp Cys Asp Ser Leu Leu Glu Pro Ser Gly
    370                 375                 380

Ala Thr Ser Gln Ala Tyr Arg Val Asp Lys Arg Ala Ala Glu Gln Ala
385                 390                 395                 400

Arg Trp Glu Glu Ala Ser Lys Glu Thr Ile Lys Lys Thr Asn Lys Pro
                405                 410                 415

Cys Pro Arg Cys Asn Val Pro Ile Glu Lys Asn Gly Gly Cys Met His
            420                 425                 430

Met Lys Cys Pro Gln Pro Gln Cys Lys Leu Glu Trp Cys Trp Asn Cys
        435                 440                 445

Gly Cys Glu Trp Asn Arg Ala Cys Met Gly Asp His Trp Phe Asp Val
    450                 455                 460

<210> 34
<211> 451
<212> PRT
<213> mouse

<400> 34
Met Ile Val Phe Val Arg Phe Asn Ser Ser Tyr Gly Phe Pro Val Glu
1               5                   10                  15

Val Asp Ser Asp Thr Ser Ile Leu Gln Leu Lys Glu Val Val Ala Lys
            20                  25                  30

Arg Gln Gly Val Pro Ala Asp Gln Leu Arg Val Ile Phe Ala Gly Lys
        35                  40                  45

Glu Leu Pro Asn His Leu Thr Val Gln Asn Cys Asp Leu Glu Gln Gln
    50                  55                  60

Ser Ile Val His Ile Val Gln Arg Pro Arg Arg Arg Ser His Glu Thr
65                  70                  75                  80

Asn Ala Ser Gly Gly Asp Glu Pro Gln Ser Thr Ser Glu Gly Ser Ile
                85                  90                  95

Trp Glu Ser Arg Ser Leu Thr Arg Val Asp Leu Ser Ser His Thr Leu
            100                 105                 110

Pro Val Asp Ser Val Gly Leu Ala Val Ile Leu Asp Thr Asp Ser Lys
        115                 120                 125

Arg Asp Ser Glu Ala Ala Arg Gly Pro Val Lys Pro Thr Tyr Asn Ser
    130                 135                 140

Phe Phe Ile Tyr Cys Lys Gly Pro Cys His Lys Val Gln Pro Gly Lys

145                 150                 155                 160

Leu Arg Val Gln Cys Gly Thr Cys Lys Gln Ala Thr Leu Thr Leu Ala
                165                 170                 175

Gln Gly Pro Ser Cys Trp Asp Asp Val Leu Ile Pro Asn Arg Met Ser
            180                 185                 190

Gly Glu Cys Gln Ser Pro Asp Cys Pro Gly Thr Arg Ala Glu Phe Phe
        195                 200                 205

Phe Lys Cys Gly Ala His Pro Thr Ser Asp Lys Asp Thr Ser Val Ala
    210                 215                 220

Leu Asn Leu Ile Thr Ser Asn Arg Arg Ser Ile Pro Cys Ile Ala Cys
225                 230                 235                 240

Thr Asp Val Arg Ser Pro Val Leu Val Phe Gln Cys Asn His Arg His
                245                 250                 255

Val Ile Cys Leu Asp Cys Phe His Leu Tyr Cys Val Thr Arg Leu Asn
            260                 265                 270

Asp Arg Gln Phe Val His Asp Ala Gln Leu Gly Tyr Ser Leu Pro Cys
        275                 280                 285

Val Ala Gly Cys Pro Asn Ser Leu Ile Lys Glu Leu His His Phe Arg
    290                 295                 300

Ile Leu Gly Glu Glu Gln Tyr Thr Arg Tyr Gln Gln Tyr Gly Ala Glu
305                 310                 315                 320

Glu Cys Val Leu Gln Met Gly Gly Val Leu Cys Pro Arg Pro Gly Cys
                325                 330                 335

Gly Ala Gly Leu Leu Pro Glu Gln Gly Gln Arg Lys Val Thr Cys Glu
            340                 345                 350

Gly Gly Asn Gly Leu Gly Cys Gly Phe Val Phe Cys Arg Asp Cys Lys
        355                 360                 365

Glu Ala Tyr His Glu Gly Asp Cys Asp Ser Leu Leu Glu Pro Ser Gly
    370                 375                 380

Ala Thr Ser Gln Ala Tyr Arg Val Asp Lys Arg Ala Ala Glu Gln Ala
385                 390                 395                 400

Arg Trp Glu Glu Ala Ser Lys Glu Thr Ile Lys Lys Thr Thr Lys Pro

                405                 410                 415

Cys Pro Arg Cys Asn Val Pro Ile Glu Lys Asn Gly Gly Cys Met His
            420                 425                 430

Met Lys Cys Pro Gln Pro Gln Cys Lys Leu Glu Trp Cys Trp Asn Cys
        435                 440                 445

Gly Cys Glu
    450